# EUROPEAN PATENT SPECIFICATION

(11) **EP 1 880 026 B1**
(45) Date of publication and mention of the grant of the patent: **13.11.2013**
(21) Application number: 06768483.7
(22) Date of filing: 15.05.2006
(51) Int. Cl.: C12Q 1/68

(54) **GENETIC POLYMORPHISMS ASSOCIATED WITH MYOCARDIAL INFARCTION AND USES THEREOF**
GENETISCHE POLYMORPHISMEN IN ZUSAMMENHANG MIT MYOKARDININFARKTEN UND IHRE VERWENDUNG
POLYMORPHISMES GÉNÉTIQUES ASSOCIÉS À UN INFARCTUS DU MYOCARDE ET LEURS UTILISATIONS

(30) Priority: 13.05.2005 KR 20050040163; 02.06.2005 KR 20050047195
(43) Date of publication of application: 23.01.2008
(73) Proprietor: Samsung Electronics Co., Ltd., Suwon-si, Gyeonggi-do, 443-742 (KR)
(72) Inventor: KIM, Byung-Chul, Hwaseong-si, Gyeonggi-do 445-779 (KR); LEE, Yeon-Su, 1003-102 Seongjeo Maeul 13-danji, Gyeonggi-do 411-704 (KR); KIM, Min-Sun, Hwaseong-si,2Gyeonggi-do 445-763 (KR); SON, Ok-Kyoung, Jongam-dong, Seoul 136-090 (KR); LEE, Moon-Soo, Seongdong-gu, Seoul 133-070 (KR); KIM, Ki-Eun, Seocho-gu, Seoul 137-786 (KR); SONG, OK-Ryul, Yeongdeungpo-gu, Seoul 150-966 (KR); JEON, Hyo-Jeong, Anyang-si, Gyeonggi-do 430-750 (KR); PARK, Kyung-Hee, Gangnam-gu, Seoul 135-280 (KR); AHN, Tae-Jin, Gangnam-gu, Seoul 135-270 (KR); KIM, Jae-Heup, Dongjak-gu, Seoul 156-775 (KR)
(74) Representative: Grünecker, Kinkeldey, Stockmair & Schwanhäusser
(86) International application number: PCT/KR2006/001812
(87) International publication number: WO 2006/121312

(56) References cited:
- WO-A-2004/031407
- WO-A1-2004/001037
- US-A1- 2004 166 491
- OZAKI KOUICHI ET AL: "Functional SNPs in the lymphotoxin-alpha gene that are associated with susceptibility to myocardial infarction. Correction of abstract.)." NATURE GENETICS, vol. 33, no. 1, January 2003 (2003-01), page 107, XP002964781 ISSN: 1061-4036
- MUKAE SHUJI ET AL: "Mitochondrial 5178A/C genotype is associated with acute myocardial infarction." CIRCULATION JOURNAL, vol. 67, no. 1, January 2003 (2003-01), pages 16-20, XP002555598 ISSN: 1346-9843
- JIANG HONG ET AL: "C/T polymorphism of the intercellular adhesion molecule-1 gene (exon 6, codon 469). A risk factor for coronary heart disease and myocardial infarction" INTERNATIONAL JOURNAL OF CARDIOLOGY, vol. 84, no. 2-3, August 2002 (2002-08), pages 171-177, XP002555599 ISSN: 0167-5273
- DATABASE EMBL [Online] 22 April 2003 (2003-04-22), "human full-length cDNA 5-PRIME end of clone CS0DI029YH23 of PLACENTA COT 25-NORMALIZED of Homo sapiens (human)" XP002555695 retrieved from EBI accession no. EMBL:BX357701 Database accession no. BX357701
- DATABASE EMBL [Online] 4 March 2004 (2004-03-04), "ij21a04.x5 Melton Normalized Human Islet 4 N4-HIS 1 Homo sapiens cDNA clone IMAGE:6135079 3' similar to TR:Q9UNA4 Q9UNA4 RAD30B. ;, mRNA sequence." XP002555696 retrieved from EBI accession no. EMBL:CK822965 Database accession no. CK822965
- DATABASE SNP [Online] XP003003957 Retrieved from NCBI Database accession no. (rs2148582)
- DATABASE SNP [Online] XP003003958 Retrieved from NCBI Database accession no. (rs5050)
- DATABASE SNP [Online] XP003003959 Retrieved from NCBI Database accession no. (rs7079)
- DATABASE SNP [Online] XP003003956 Retrieved from NCBI Database accession no. (rs699)
- HAGA H. ET AL.: 'Gene-based SNP discovery as part of the Japanese Millenium Genome Project' JOURNAL OF HUMAN GENETICS vol. 47, no. 11, November 2002, pages 605 - 610, XP002278549
- RANJITH N. ET AL.: 'Renin-angiotensin system and associated gene polymorphisms in myocardial infarction' CARDIOVASCULAR JOURNAL OF SOUTHERN AFRICA vol. 15, no. 1, January 2004 - February 2004, pages 22 - 26, XP003008860
- HALUSHKA M.K. ET AL.: 'Patterns of SNPs in candidate genes for blood-pressure homeostasis' NATURE GENETICS vol. 22, no. 3, July 1999, pages 239 - 247, XP002906054
- [Online] 04 October 2002, Retrieved from the Internet: <URL:http://www.ncbi.nlm.nih.gov/projects/S NP/snp_ref.cgi?rs=3730668>

## Description

### Technical Field

The present invention relates to genetic polymorphisms associated with myocardial infarction and uses thereof.

### Background Art

99.9% of base sequences of the human genome are identical. Diversity in individuals' appearance, behavior and susceptibility to certain diseases is caused by partial differences in the remaining 0.1% of the base sequences in the human genome. That is, differences in about 3 million base sequences of the human genome account for the diversity among individuals, communities, races and peoples. The differences in base sequences contribute to the differences in disease distributions as well as phenotypic distinctions such as skin color of different races. There are about 3 billion base pairs, and base pairs in which polymorphisms occur are positioned at intervals of about 1.0kb. There are about 3 million base pairs in total that vary from person to person, and variations in these base pairs are referred to as Single Nucleotide Polymorphisms (SNPs). Causes of the diversity among individuals and communities or the difference between a disease group and a normal group may be found through the analysis of the 3 million SNPs, and analysis of entire base sequences is unnecessary.

The prime object of genetics is to map phenotypic differences such as diseases in humans to variations in DNA. A polymorphic marker existing in all genomes is the best means of obtaining this object. Microsatellite markers have been commonly used as polymorphic markers to distinguish individuals and find genes related to genetic diseases, but SNPs have drawn attention with the development of DNA chips. Automatic SNP detection on a large scale is possible because of the high frequency of SNPs, the safety of using SNPs, and the even distribution of SNPs across all genomes. SNPs will contribute to predictive medical science, which is a new branch of medical science. For example, a revolutionary process of predicting diseases of individuals and investigating individual's reactions to a certain medical supply can be performed by implementing up-to-date biotechnology such as a DNA chip technique and a high speed DNA sequence analysis technique.

The study of SNPs involves an analysis of genotypes evenly distributed throughout the population. By studying SNPs, a population may be divided according to genotype, and if a disease group is significantly distributed according to a genotype, the relationship between the genotype and the disease can be established. In most studies of SNPs, if a single genotype or several genotypes have significantly different distributions in a disease group and a normal group, the differences in disease frequency according to the genotype may be analyzed.

About 510,000 SNPs, approximately one sixth of the 3 million SNPs in human genomes, exist in genes. It is very important to know the distribution of such SNPs in genes because the SNPs are directly related to gene expression or protein functions. If a genotype associated with a certain disease can effect a change in a gene expression or a protein function, the gene or the protein is likely to be a cause of the disease. In this case, the gene can be the target gene for disease detection and treatment. The susceptibility to the disease may also be analyzed using SNP analysis of the gene.

SNPs in transcription regulatory regions of a gene sequence, such as a promoter, can regulate the quantity of expressed genes. On rare occasions, SNPs also influence the stability and translation efficiency of RNA located in sequences at exon-intron boundaries affecting RNA splicing or in a 3'-untranslated region (3'-UTR).

That is, the SNPs that are located in an encoding region or a transcription and translation regulatory region may be a useful index for determining susceptibility to a disease. Although some SNPs are located in an encoding region or a transcription and translation regulatory region, other SNPs which are not expected to affect protein expression and/or function could be found associated with a disease. International researches are being tried to reveal the the role of such SNPs in human diseases.

Genotype analysis using haplotypes provides more accurate results since haplotypes have more information than SNPs and contain information about linkage disequilibrium. Particularly, when several SNPs are densely distributed in a gene, the gene analysis using haplotypes, which have combined information regarding adjacent SNPs, is effective for finding the relation between a gene function and a disease. For example, a haplotype of Five Lipo-oxygenase Activation Pepetide (FLAP) has been found to be a more accurate marker for a heart attack than a SNP, and has been reported to be a critical cause of heart attacks (Helgadotiir et al., Nat Genet. 2004 Mar; 36(3):233-9). Since then, Decode Genetics has performed research on an inhibitor of the enzyme containing FLAP haplotypes for preventative medicine for heart attacks based on the reported results of the haplotype, and a second phase clinical trial has already commenced.

Meanwhile, cardiovascular disease is a major cause of death in industrialized countries around the world, and has been a major cause of death in the Republic of Korea since the 1970s. According to the Korean National Statistical Office, in 2003, 22,000 out of 246,000 deaths (9087 per 100,000, or 9.1 %) were the result of cardiac disorders and hyperpiesia, which are the third leading cause of death in Korea following cancer and cerebrovascular disease.

Coronary artery disease, which ranks high among cardiovascular diseases, is usually caused by arteriosclerosis, the blocking or narrowing of coronary arteries supplying blood to the heart. Blocking of the coronary artery indicates myocardial infarction and narrowing of the coronary artery indicates angina pectoris. The causes of coronary artery disease are known to be hyperlipidemia (hypercholesterolemia), hyperpiesia, smoking, diabetes, genetic inheritance, obesity, lack of exercise, stress and menopause. A subject having complex factors of coronary artery disease has a higher risk of incidence.

### Disclosure of Invention

### Technical Problem

Currently, X-ray and ultrasonography of the interior of the heart and coronary artery can be used for cardiovascular disease diagnosis. However, as with the diagnosis or prognosis of other various cardiovascular and complicated diseases including myocardial infarction using other physical techniques, the diagnosis or prediction can be performed only when the diseases are at an advanced stage.

### Technical Solution

SUMMARY OF THE INVENTION

The present inventors found SNPs as a result of research to find SNPs associated with myocardial infarction, which makes it possible to predict the incidence probability of and genetic susceptibility to myocardial infarction.

The present invention provides a single nucleotide polymorphism (SNP) associated with myocardial infarction.

The present invention also provides a polynucleotide specifically hybridized with the polynucleotide containing the SNP.

The present invention also provides a polypeptide encoded by the polynucleotide.

The present invention also provides an antibody specifically bound to the polypeptide.

The present invention also provides a microarray for detecting SNPs including the polynucleotide.

The present invention also provides a kit for detecting SNPs including the polynucleotide.

The present invention also provides a method of identifying a subject having a changed risk of incidence of myocardial infarction.

The present invention also provides a method of detecting SNPs in nucleic acid molecules.

The present invention also provides a method of screening pharmaceutical compositions for myocardial infarction.

The present invention also provides a method of regulating gene expression.

According to an aspect of the present invention, there is provided a polynucleotide of nucleotide sequences of SEQ ID NOS: 1 to 60 and 241 to 244 including at least 8 contiguous nucleotides and the 101^{st} base of the nucleotide sequence and complementary polynucleotides of the nucleotide sequences.

According to another aspect of the present invention, there is provided a polynucleotide hybridized with the polynucleotide or the complementary polynucleotide thereof.

According to another aspect of the present invention, there is provided a polypeptide encoded by the polynucleotide.

According to another aspect of the present invention, there is provided an antibody specifically bound to the polypeptide.

According to another aspect of the present invention, there is provided a microarray for detecting SNPs including the polynucleotide, the polypeptide encoded by the polynucleotide or cDNA of the polynucleotide.

According to another aspect of the present invention, there is provided a kit for detecting SNPs including the polynucleotide, the polypeptide encoded by the polynucleotide or cDNA of the polynucleotide.

According to another aspect of the present invention, there is provided a method of identifying a subject having a changed risk of incidence of myocardial infarction including isolating a nucleic acid sample from the subject and determining an allele at a polymorphic site of one or more polynucleotides among SEQ ID NOS: 1 to 60 and 241 to 244, wherein the polymorphic site is positioned at the 101^{st} nucleotide of the polynucleotides.

According to another aspect of the present invention, there is provided a method of detecting SNPs in nucleic acid molecules including contacting a test sample having nucleic acid molecules with a reagent specifically hybridized under strict conditions with a polynucleotide of nucleotide sequences of SEQ ID NOS: 1 to 60 and 241 to 244 containing at least 8 contiguous nucleotides and the 101^{st} base of the nucleotide sequence and complementary polynucleotides of the nucleotide sequences and detecting the formation of a hybridized double-strand.

According to another aspect of the present invention, there is provided a method of screening pharmaceutical compositions for myocardial infarction including contacting a candidate material with a polypeptide encoded by a polynucleotide of nucleotide sequences of SEQ ID NOS: 1 to 60 and 241 to 244 containing at least 8 contiguous nucleotides and the 101^{st} base of nucleotide sequence and complementary polynucleotides of the nucleotide sequences under proper conditions for the formation of a binding complex and detecting the formation of the binding complex between the polypeptide and the candidate material.

According to another aspect of the present invention, there is provided a method of regulating gene expression including binding an anti-sense nucleotide or Si RNA with a polynucleotide of nucleotide sequences of SEQ ID NOS: 1 to 60 and 241 to 244 containing at least 8 contiguous nucleotides and the 101^{st} base of the nucleotide sequence, wherein the anti-sense nucleotide and Si RNA is specific to the polynucleotide.

The above aspects and advantages of the present invention will become more apparent by describing in detail exemplary embodiments thereof.

DETAILED DESCRIPTION OF THE INVENTION

A single nucleotide polymorphism (SNP) associated with myocardial infarction according to an aspect of the present invention includes a polynucleotide of nucleotide sequences of SEQ ID NOS: 1 to 60 and 241 to 244 containing at least 8 contiguous nucleotides and the 101^{st} base of the nucleotide sequence, and complementary polynucleotides of the nucleotide sequences.

A polynucleotide containing one of SEQ ID NOS: 1 to 60 and 241 to 244 is a polymorphic sequence. A polymorphic sequence is a nucleotide sequence containing polymorphic sites where SNPs exist in a polynucleotide sequence. The polynucleotide may be DNA or RNA.

Herein, the SNP may be the most commonly found single base-pair variation among DNA sequence polymorphisms shown in about every 1 kb in the DNA of individuals.

In Examples of the present invention, a series of selections were made in order to find SNPs closely associated with cardiovascular disease, more particularly with myocardial infarction. DNA was isolated from blood of myocardial infarction patients and normal persons and amplified. After an analysis of the SNP sequence in the DNA, SNPs having very different appearance frequencies between the patients and normal persons were identified. 64 SNPs and the genotypes thereof which were identified in Examples are disclosed in Table 1 to 3.

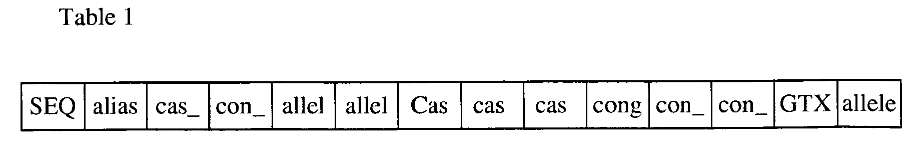

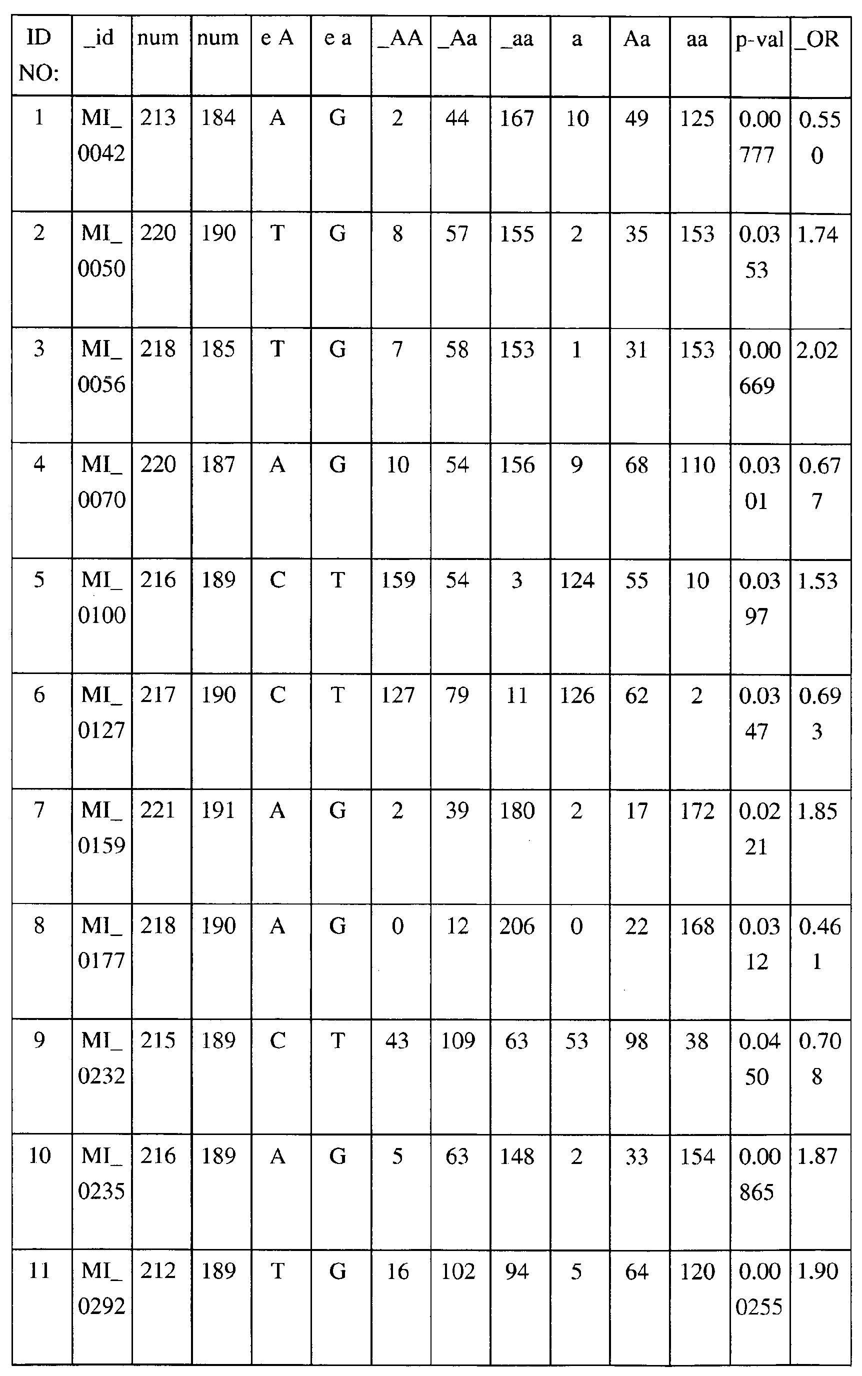

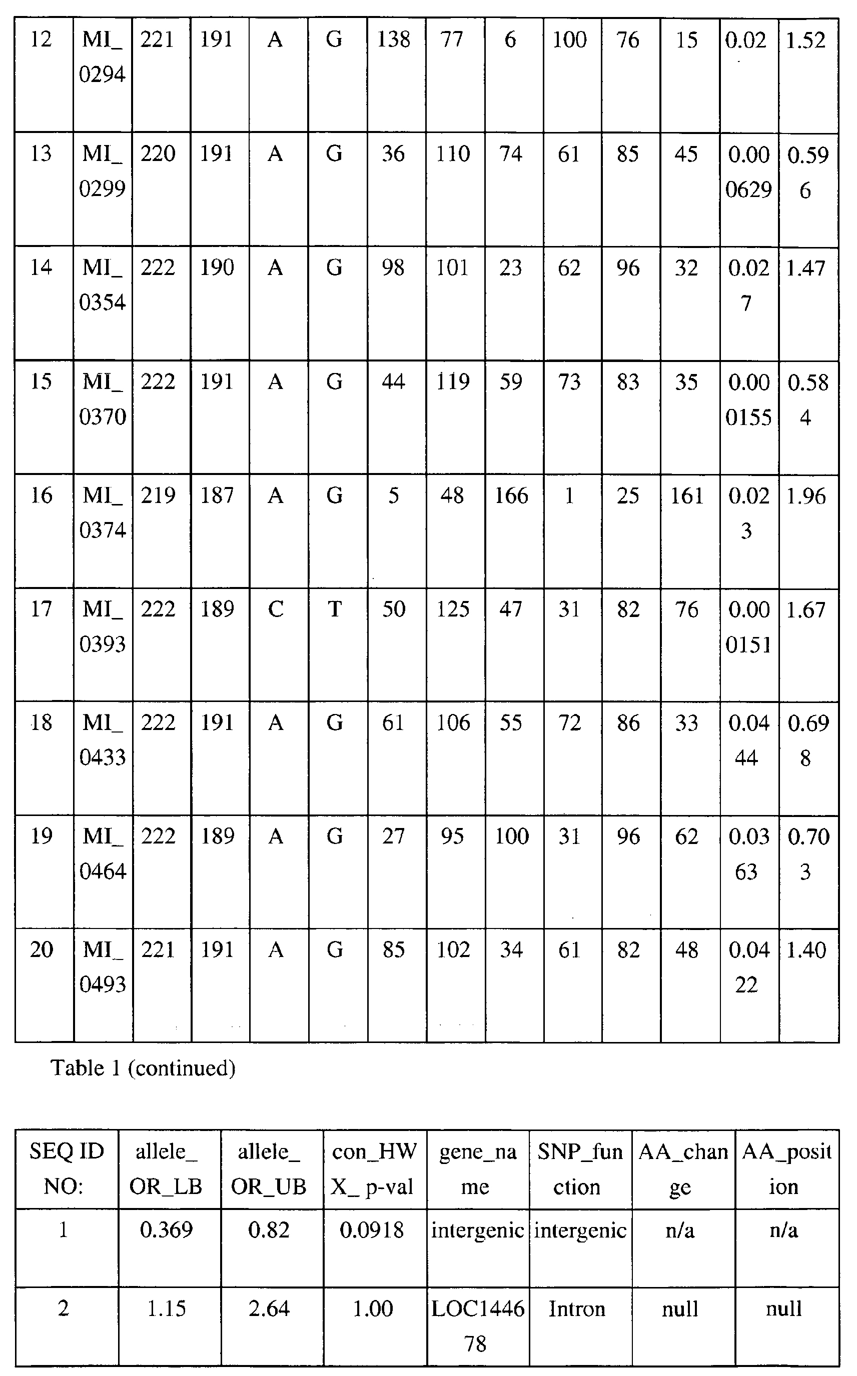

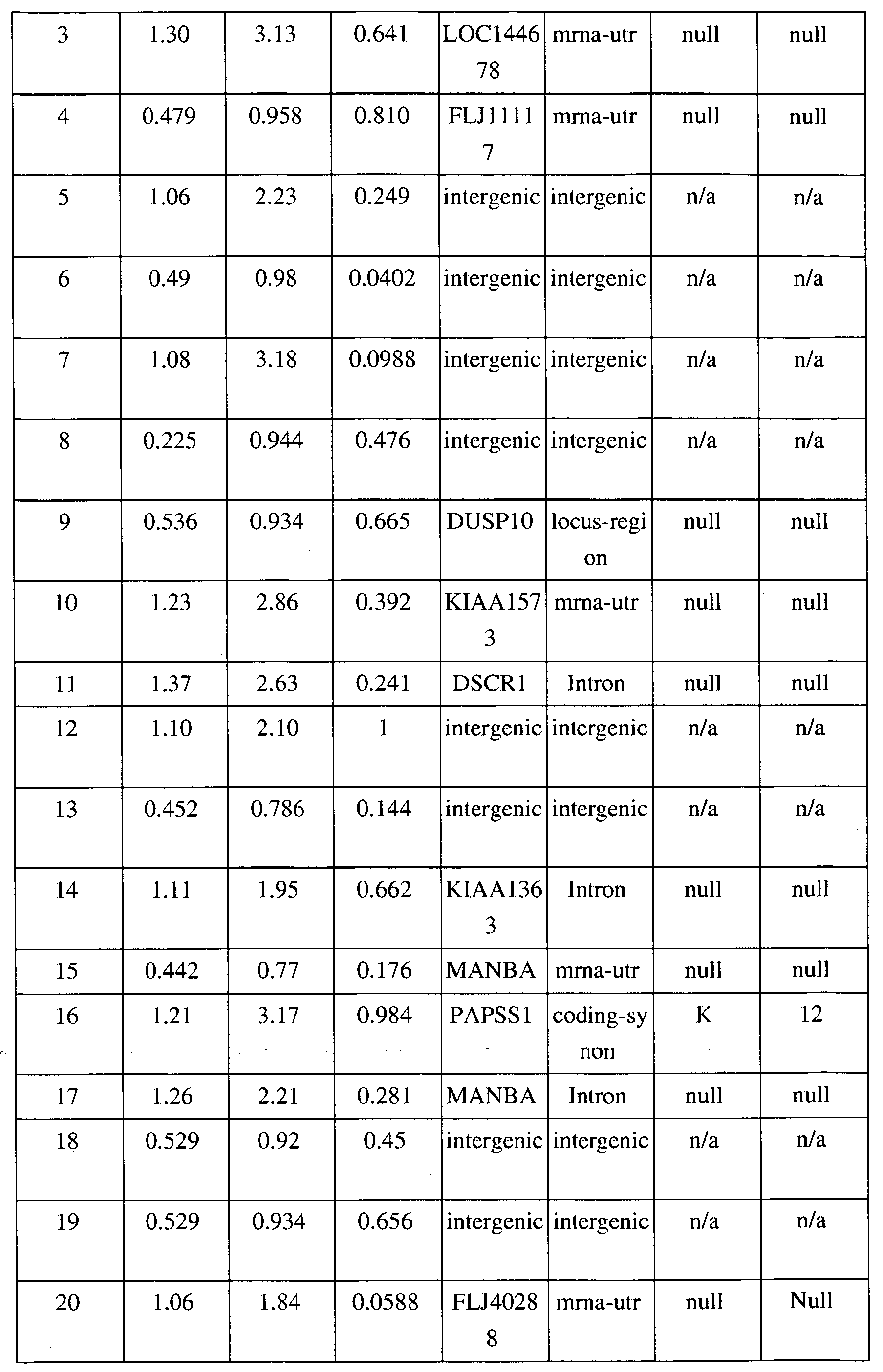

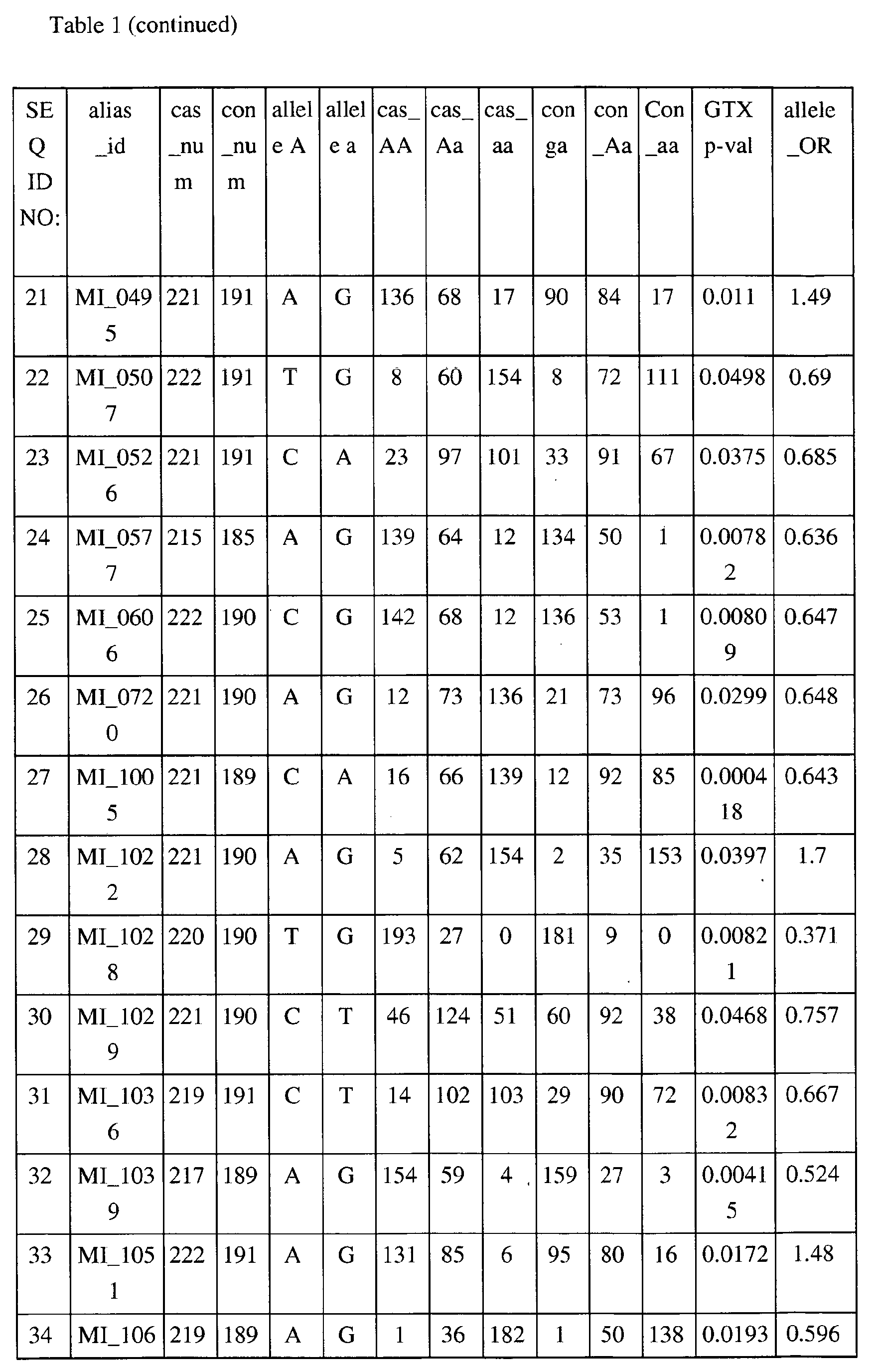

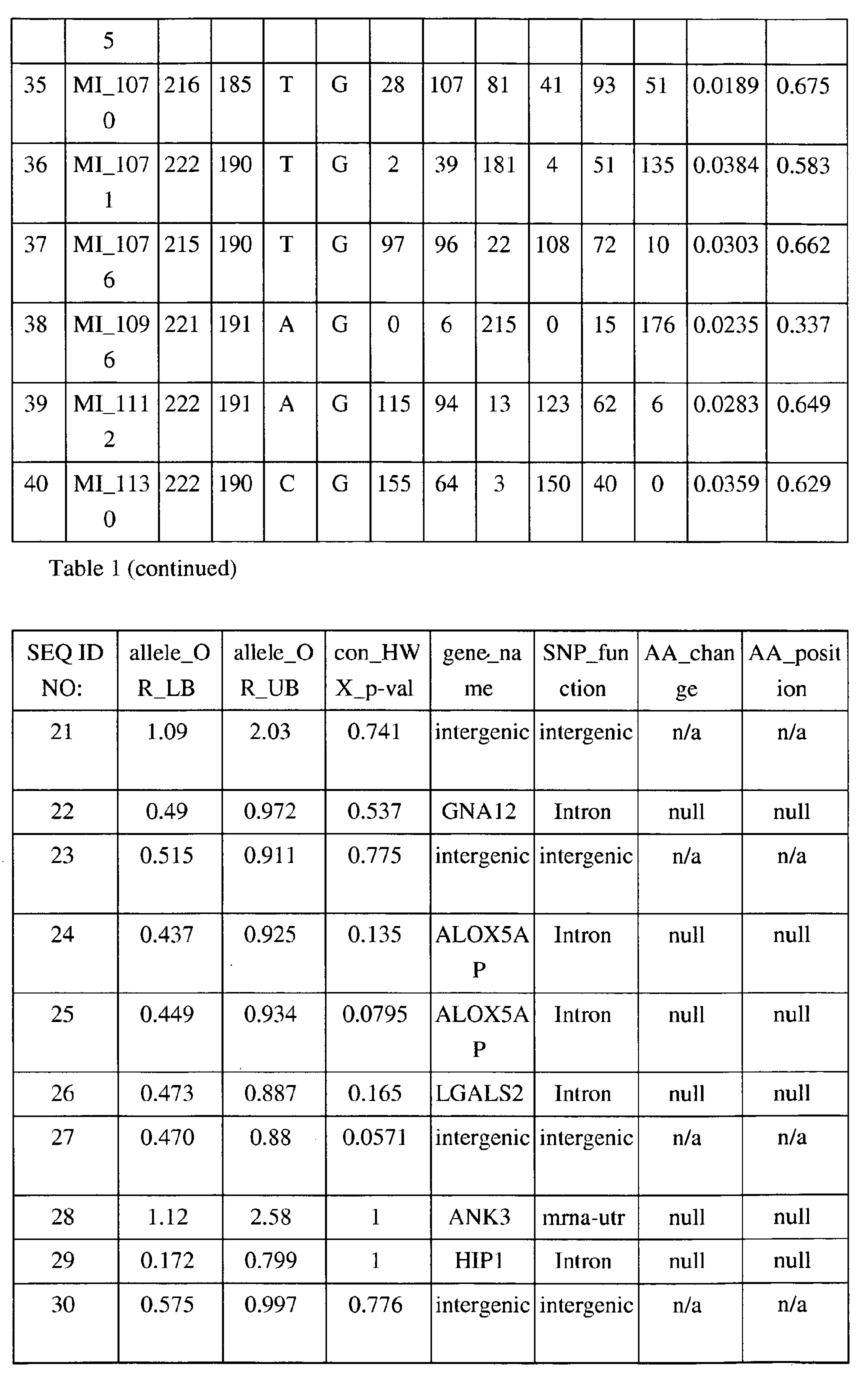

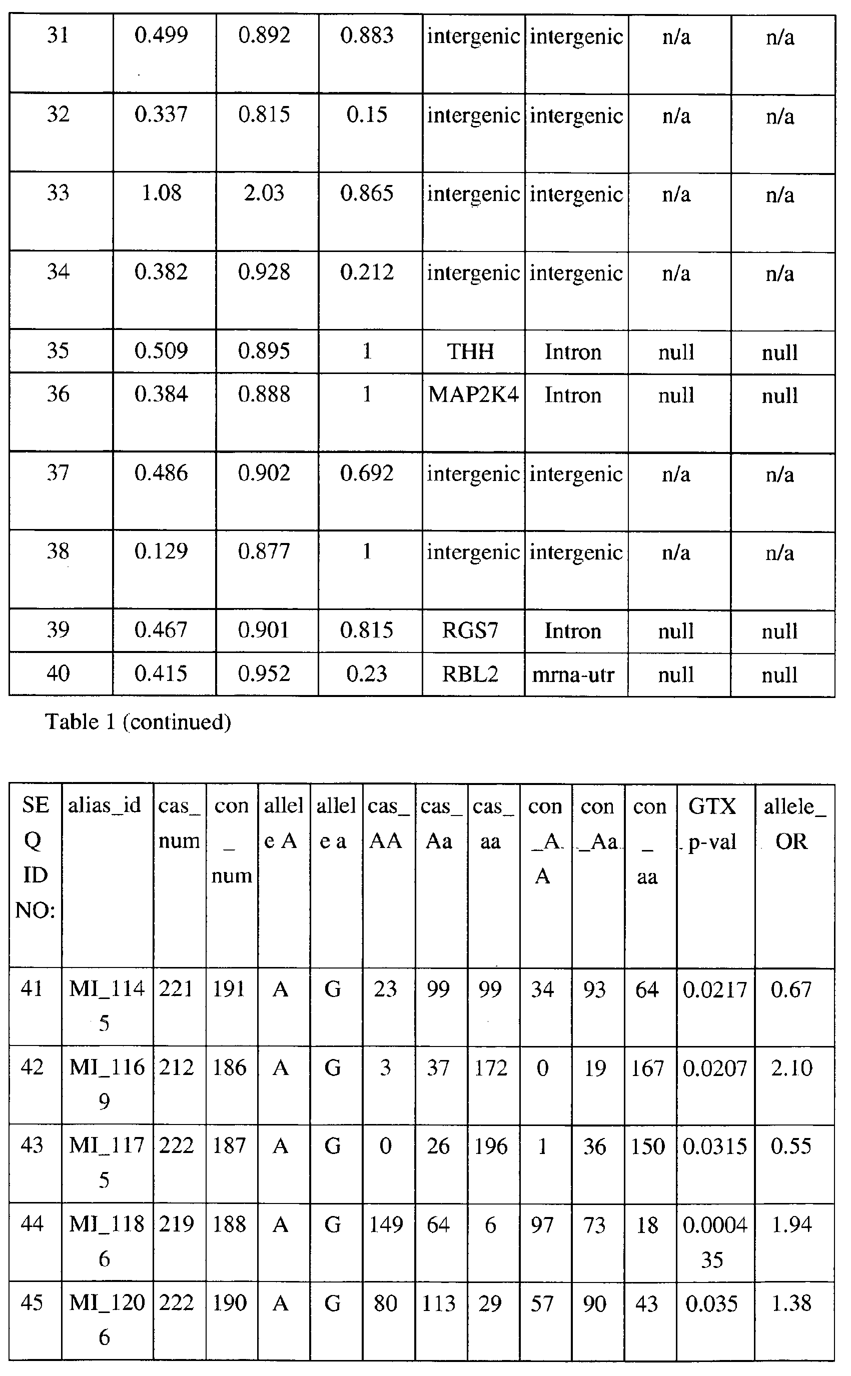

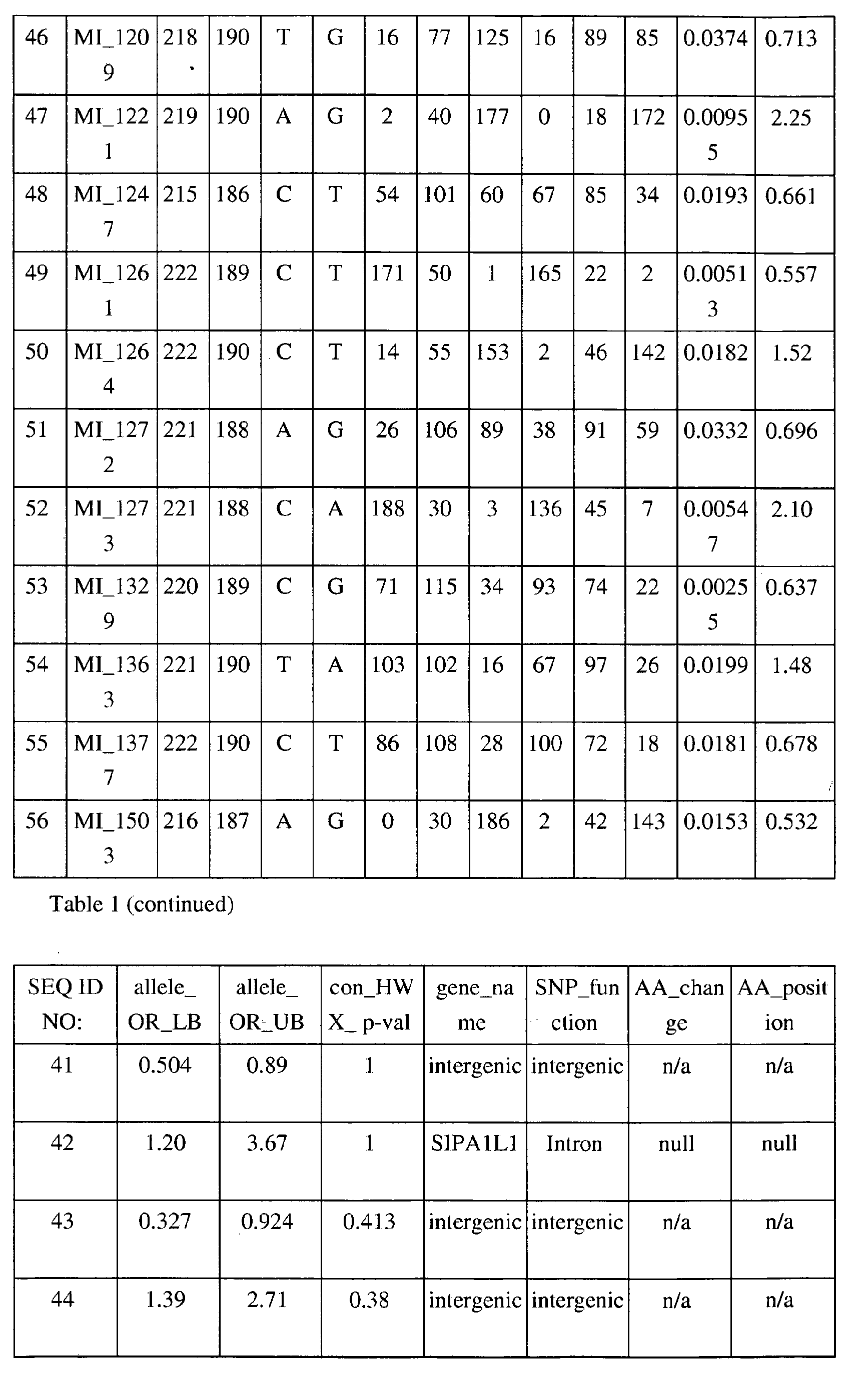

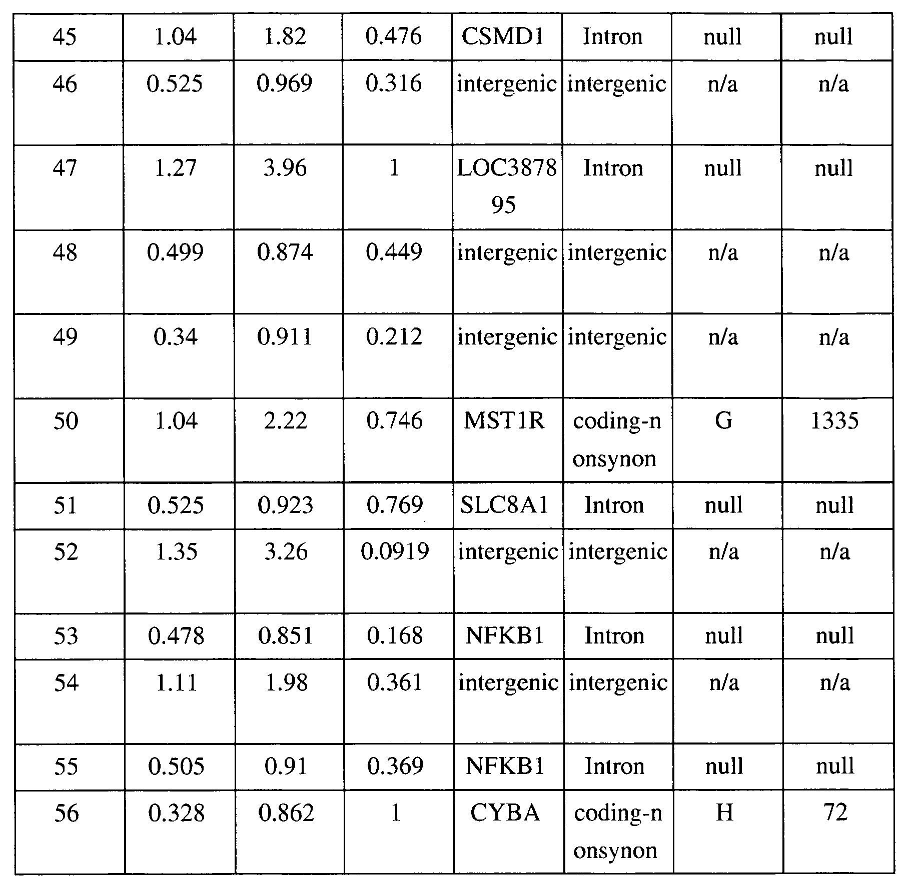

[43]

In Table 1 and 2, 'SEQ ID NO: ' is the polynucleotide sequence identification number including the SNP enclosed with the specification of the present invention.

'Alias_id' is a SNP number arbitrarily designated by the inventors of the present invention.

'Cas_num' and 'con_num' respectively indicate the number of patients and normal persons having the SNP.

Allele 'A' and 'a' respectively represent a low mass allele and a high mass allele in sequencing experiments according to a homogeneous MassEXTEND™ technique of Sequenom, and are arbitrarily designated for convenience of experiments.

'Cas AA', 'cas_Aa' and 'cas_aa' respectively represent the number of patients having the genotype 'AA', 'Aa' and 'aa'. Also, 'con_AA', 'con_Aa' and 'con_aa' respectively represent the number of normal persons having the genotype 'AA', 'Aa' and 'aa'.

'GTX_p-val' is the p-value obtained by inspecting the gene using Fisher's exact test. When the p-value was 0.05 or less, it was determined that the genotype between the disease group and the normal group was not identical, i.e. significant.

'Allele_OR' is the odds ratio of the probability of the SNP in the disease group to the probability of the SNP in the normal group based on genotype.

'Allele_OR_LB' and 'allele_OR_UB' respectively indicate the lower limit and the upper limit of the 95% confidence interval of the odds ratio. When the odds ratio exceeds 1, 'A' is the risk factor and when the odds ratio is less than 1, 'a' is the risk factor. When the confidence interval includes 1, it cannot be determined that the relationship between the genotype and the disease is significant.

'Con_HWX_p-val' indicates the Hardy-Weinberg Equilibrium in the normalgroup. When the p-value was 0.05 or less, the normal group was not determined to be in Hardy-Weinberg Equilibrium.

'Gene_name' is the name of the gene to which the SNP belongs.

'SNP_function' is the location of the SNP within the gene.

'AA_change' indicates whether an amino acid is changed by the SNP.

'AA_position' indicates a position in the polypeptide of an amino acid coded by the SNP site.

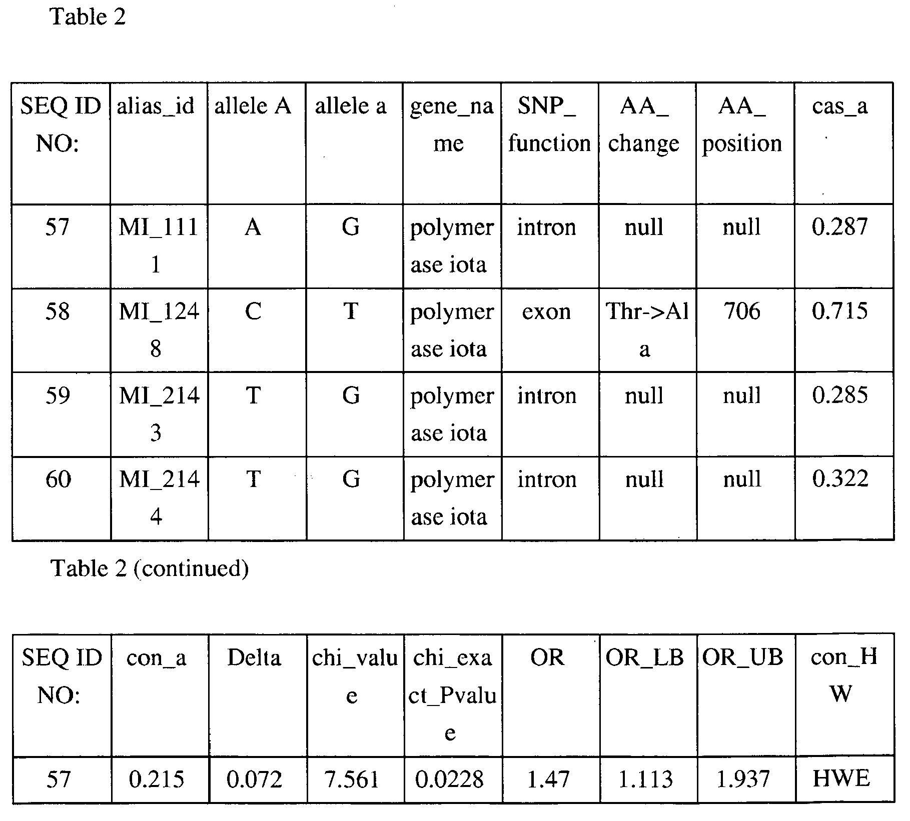

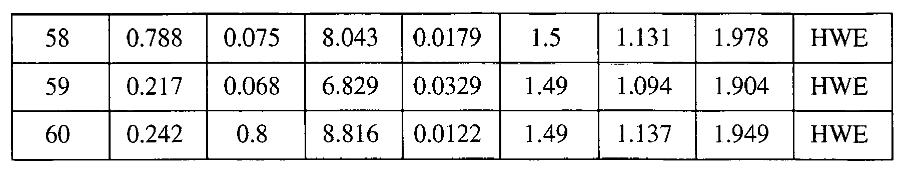

Table 3

| SEQ ID NO: | GenBank accession No. of SNP in NCBI | allele A | allele a | OR | OR_LB | OR_UB | Chi_exact_ p-Value |
|---|---|---|---|---|---|---|---|
| 241 | rs214858 2 | A | G | 0.619 | 0.436 | 0.877 | 0.0296 |
| 242 | rs5050 | T | G | 0.659 | 0.455 | 0.955 | 0.0281 |
| 243 | rs7079 | T | G | 0.657 | 0.428 | 1.01 | 0.0583 |
| 244 | rs699 | G | A | 1.61 | 1.14 | 2.29 | 0.0297 |

'Cas_a', 'con_a' and 'Delta' respectively indicate the frequency of 'a' in the disease group, the frequency of 'a' in the normal group, and the absolute value of the difference between 'cas_a' and 'con_a'. Herein, 'cas_a' is given by (the frequency of the genotype 'aa' × 2 + the frequency of the genotype 'Aa')/(the number of samples of the disease group × 2) and 'con_a' is given by (the frequency of the genotype 'aa' x 2 + the frequency of the genotype 'Aa')/(the number of samples of the normal group x 2)

'Chi-value' is obtained through a chi-square test, and was used for p-value calculation. 'Chi-exact-p-value' indicates the p-value of Fisher's exact test of chisqueare test, and is a variable used to determine statistical significance more accurately, since the chi-square test result may be inaccurate when the number of genotypes is less than 5. When the p-value was 0.05 or less, it was determined that the genotype between the disease group and the normal group was not identical, i.e. significant.

'OR' is a ratio noticing how often a specific genotype is found in a group of the disease group and the normal group and is calculated as (the number of patients having a specific genotype in the disease group) × (the number of persons not having the specific genotype in the normal group)/(the number of patients not having the specific genotype in the disease group) × (the number of persons having the specific genotype in the normal group). 'OR_LB', 'OR_UB' respectively indicate the minimum value and the maximum value of the confidence interval of OR at a 5% level of significance.

Four SNPs in table 3 are all located in the AGT gene.

A haplotype for diagnosis of myocardial infarction according to an aspect of the present invention may be composed of polynucleotides of SEQ ID NOS: 57 to 60. The SNPs' linkage disequlibriums (LD) with each other are disclosed in Table 4. As illustrated in Table 4, the four SNPs composed a strong LD block,

**Table 4**

| | MI_2144 | MI_1111 | MI_2143 | MI_1248 |
|---|---|---|---|---|
| MI_2144 | 0 | 1 | 1 | 0.9898 |
| MI_1111 | 1 | 0 | 1 | 0.9951 |
| MI_2143 | 1 | 1 | 0 | 1 |
| MI_1248 | 0.9898 | 0.9951 | 1 | 0 |

For example, the 101^{st} base, which is the SNP site, of the polynucleotides of SEQ ID NOS: 57 to 60 composing the haplotype can be the risk allele. That is, the haplotype can be a haplotype No. 1 or 2 in Table 5.

**Table 5**

| Haplotype No. | MI_2144 | MI_1111 | MI_2143 | MI_1248 | Hap.score | p.val | Hap.Freq total_free |
|---|---|---|---|---|---|---|---|
| 1 | A | A | A | a | -3.06506 | 0.00218 | 71.60% |
| 2 | a | a | a | A | 2.71413 | 0.00664 | 25.00% |
| 3 | a | A | A | a | 0.73651 | 0.46142 | 3% |

'A' and 'a' in Table 5 indicate alleles. For example, the haplotype No. 1 is a haplotype including four SNPs at which the alleles are 'A', 'A', 'A' and 'a'. 'Hap.score' shows how well the haplotype can classify subjects into the normal group and the disease group. When the 'p.val' was 0.05 or less, it was determined that the relationship between the genotype and the disease was significant.

Alternatively, the haplotype for diagnosis of myocardial infarction according to an embodiment of the present invention may be composed of the polynucleotides of SEQ ID NOS: 241 to 244.

The 101^{st} base, which is the SNP site, of the polynucleotides of SEQ ID NOS: 241 to 244 composing the haplotype can be the risk allele. That is, the haplotype can be a haplotype No. 4 or 7 in Table 6.

**Table 6**

| Haplotype No. | rs21485 82 | rs5050 | rs7079 | rs699 | p-value | Hap.Freq total_freq | y.0 con_fre q | y.1 cas_freq |
|---|---|---|---|---|---|---|---|---|
| 4 | A | A | A | a | 0.024 | 0.110 | 0.135 | 0.088 |
| 5 | A | A | a | a | 0.134 | 0.084 | 0.099 | 0.071 |
| 6 | a | A | a | A | 0.608 | 0.627 | 0.618 | 0.634 |
| 7 | a | a | a | A | 0.035 | 0.172 | 0.142 | 0.198 |

'Hap.Freq total_freq' indicates the frequency of the haplotype in the disease group and the normal group.

'Y.0 con_freq' indicates the frequency of haplotype in the normal group.

'Y.1 cas_freq' indicates the frequency of haplotype in the disease group.

Predicting the incidence of cardiovascular disease using only a genetic factor is difficult since the occurrence of cardiovascular disease may be affected by various environmental or habitual factors. SNPs which are significantly associated with a subject having certain characteristics were selected. In addition, SNPs which exist in the patients with cardiovascular disease but do not exist in the normal persons were identified.

It was that male non-smokers showed a more significant relationship between the existence of the SNPs of a nucleotide sequence of SEQ ID NOS: 57 to 60 and the incidence of myocardial infarction. This was proved by the fact that the male non-smoker had an increased odds ratio in Table 7. Therefore, according to an embodiment of the present invention, the SNP for diagnosis of myocardial infarction may be a nucleotide sequence of SEQ ID NOS: 57 to 60 and the subject may be a male non-smoker.

**Table 7**

| group | alias_id | SEQ ID NO: | OR | OR_LB | OR_UB | Chi_squ_P value |
|---|---|---|---|---|---|---|
| Male | MI_1111 | 57 | 1.3816 | 1.0067 | 1.8961 | 0.0939 |
| | MI_1248 | 58 | 1.4353 | 1.0427 | 1.9757 | 0.0674 |
| | MI_2143 | 59 | 1.3513 | 0.98 | 1.8587 | 0.112 |
| | MI_2144 | 60 | 1.4705 | 1.08 | 2 | 0.0547 |
| Male smoker | MI_1111 | 57 | 1.8472 | 1.0138 | 3.3657 | 0.161 |
| | MI_1248 | 58 | 1.8752 | 1.029 | 3.4173 | 0.166 |
| | MI_2143 | 59 | 1.8181 | 1.033 | 3.1746 | 0.122 |
| | MI_2144 | 60 | 1.8518 | 1.0141 | 3.3557 | 0.132 |
| Male non-smoker | MI_1111 | 57 | 2.2865 | 1.2005 | 4.3546 | 0.0231 |
| | MI_1248 | 58 | 2.3976 | 1.2492 | 4.6018 | 0.024 |
| | MI_2143 | 59 | 2.2727 | 1.2004 | 4.3478 | 0.0242 |
| | MI_2144 | 60 | 2.5641 | 1.3458 | 4.7619 | 0.0107 |

The polynucleotide according to an embodiment of the present invention may be a nucleotide sequence of SEQ ID NO: 241, 243 or 244 and may be used for the diagnosis of myocardial infarction of a subject having a high C-reactive protein (CRP) level.

The CRP level of blood indicates the degree of inflammation, and is used to measuring the risk of cardiovascular disease. While the CRP level of female subjects in menopause who had cardiovascular disease was 0.42mg/dl, the CRP level of female subjects in menopause who did not have cardiovascular disease was 0.28mg/dl (Ridker PM, Hennekens CH, Buring JE, Rifai N., C-reactive protein and other markers of inflammation in the prediction of cardiovascular disease in women, N Engl J Med, 2000, 342:836-843).

In a subject having a polynucleotide of nucleotide sequences of SEQ ID NOS: 241, 243 and 244, the CRP level is not limited since it can be estimated that the subject having a higher CRP level has a relatively higher probability of incidence of cardiovascular disease.

**Table 8**

| SEQ ID NO: | GenBank accession No. of SNP in NCBI | Risk allele | Odds Ratio | Confidence interval | Chi_exact_p Value |
|---|---|---|---|---|---|
| 241 | rs2148582 | G | 0.276 | (0.118, 0.645) | 0.0052 |
| 243 | rs7079 | G | 0.348 | (0.121, 0.999) | 0.0485 |
| 244 | rs699 | G | 3.45 | (1.48, 8.04) | 0.0101 |

The polynucleotide according to an embodiment of the present invention may be a nucleotide sequence of SEQ ID NO: 241, 243 or 244 or a complementary polynucleotide thereof, and may be used for the diagnosis of cardiovascular disease of young subjects.

In connection with myocardial infarction, men over 45 and women over 55 who have entered menopause are known to be risk groups.

In a subject having a polynucleotide of nucleotide sequences of SEQ ID NOS: 241, 243 and 244, the age is not limited since it can be estimated that a younger subject has a relatively lower probability of incidence of cardiovascular disease.

**Table 9**

| SEQ ID NO: | GenBank accession No. of SNP in NCBI | Risk allele | Odds Ratio | Confidence interval | Chi_cxact_p Value |
|---|---|---|---|---|---|
| 241 | Rs2148582 | G | 0.482 | (0.28, 0.83) | 0.0307 |
| 243 | rs7079 | G | 0.345 | (0.169, 0.706) | 0.0058 |
| 244 | rs699 | G | 2.04 | (1.19, 3.52) | 0.0309 |

The polynucleotide according to an embodiment of the present invention may be a nucleotide sequence of SEQ ID NO: 241, 242 or 244 or a complementary polynucleotide thereof, and may be used for the diagnosis of cardiovascular disease of subjects not having diabetes.

Table 10 contains the results obtained from Examples of the present invention in which subjects do not have diabetes.

**Table 10**

| SEQ ID NO: | GenBank accession No. of SNP in NCBI | Risk allele | Odds Ratio | Confidence interval | Chi_exact_p Value |
|---|---|---|---|---|---|
| 241 | rs2148582 | G | 0.622 | (0.433, 0.892) | 0.0378 |
| 242 | rs5050 | G | 0.641 | (0.434, 0.946) | 0.0141 |
| 244 | rs699 | G | 1.6 | (1.12, 2.3) | 0.0395 |

The polynucleotide according to an embodiment of the present invention may be a nucleotide sequence of SEQ ID NO: 241, 242 or 244 or a complementary polynucleotide thereof, and may be used for the diagnosis of cardiovascular disease of subjects that smoke.

Table 11 contains the results obtained from Examples of the present invention in which subjects smoke.

**Table 11**

| SEQ ID NO: | GenBank accession No. of SNP in NCBI | Risk allele | Odds Ratio | Confidence interval | Chi_exact_p Value |
|---|---|---|---|---|---|
| 241 | rs2148582 | G | 0.545 | (0.317, 0.938) | 0.0392 |
| 242 | rs5050 | G | 0.465 | (0.236, 0.914) | 0.0458 |
| 244 | rs699 | G | 1.83 | (1.07, 3.16) | 0.0392 |

The polynucleotide according to an embodiment of the present invention may be SEQ ID NO: 243 or a complementary polynucleotide thereof, and may be used for the diagnosis of cardiovascular disease of subjects having a high TG level.

As a result of an 8-year prospective epidemiological study of the Prospective Cardiovascular Münster (PROCAM), it was discovered that the TG level during an empty stomach is quantitively relative to the frequency of cardiovascular disease. When a subject had a quite high TG level, for example 400-799 mg/dl, it was found that the incidence rate of the cardiovascular disease increased more than three times relative to the normal TG level.

In a subject having the polynucleotide of SEQ ID NO: 243, the TG level is not limited since a subject showing a high TG level has a relatively higher probability of incidence of cardiovascular disease.

**Table 12**

| SEQ ID NO | GenBank accession No. of SNP in NCBI | Risk allele | Odds Ratio | Confidence interval | Chi_exact_p Value |
|---|---|---|---|---|---|
| 243 | rs7079 | G | 0.167 | (0.0341, 0.814) | 0.035 |

In an embodiment of the present invention, a polynucleotide containing a SNP may include at least 8 contiguous nucleotides, for example, 8 to 70 of the contiguous nucleotides.

In an aspect of the present invention, a polynucleotide is specifically hybridized with a polynucleotide of SEQ ID NOS: 1 to 60 and 241 to 244 or a complementary polynucleotide thereof. The polynucleotide may be allele-specific.

The polynucleotide may include at least 8 contiguous nucleotides, for example, 8 to 70 of the contiguous nucleotides.

The allele-specific polynucleotide is a polynucleotide specifically hybridized with each allele base of the polynucleotide. The hybridization can be performed to specifically distinguish the bases in polymorphic sites among polymorphic sequences of SEQ ID NOS: 1 to 60 and 241 to 244. The hybridization can be carried out under strict conditions, for example, in a salt concentration of 1 M or less and at a temperature of 25 °C or higher. For example, 5 × SSPE (750 mM NaCl, 50 mM Na Phosphate, 5 mM EDTA, pH 7.4) and 25 to 30 °C may be suitable conditions for the allele-specific probe hybridization.

In an embodiment of the present invention, the allele-specific polynucleotide can be a primer. The primer is a single-strand oligonucleotide capable of initiating template-directed DNA synthesis in an appropriate buffer under appropriate conditions, for example, in the presence of four different nucleotide triphosphates and a polymerizing agent such as DNA, RNA polymerase or reverse transcriptase at a proper temperature. The length of the primer may vary according to the purpose of use, but is 15 to 30 nucleotides in an embodiment of the present invention. A short primer molecule can require a lower temperature to be stably hybridized with the template. The primer sequence does not necessarily need to be completely complementary to the template, but can be sufficiently complementary to be hybridized with the template. The 3' end of the primer is arranged to correspond to the polymorphic sites of SEQ ID NOS: 1 to 60 and 241 to 244. The primer is hybridized with the target DNA including the polymorphic site and initiates amplification of an allele having complete homology to the primer. The primer and another primer hybridized with the other side are used as a primer pair. Amplification is performed from the two primers, indicating that there is a specific allele in the polynucleotide. According to an embodiment of the present invention, the primer includes a polynucleotide fragment used in a ligase chain reaction (LCR). For example, the primer may be a polynucleotide used in the Examples below.

In an embodiment of the present invention, an allele specific polynucleotide may be a probe. The probe is a hybridization probe, which is an oligonucleotide capable of binding specifically to a complementary strand of a nucleic acid. Such a probe includes a peptide nucleic acid introduced by Nielsen et al., Science 254, 1497-1500 (1991). According to an embodiment of the present invention, the probe is an allele-specific probe. When a polymorphic site is located in nucleic acid fragments derived from two members of the same species, the allele-specific probe can hybridize with the DNA fragment derived from one member but not with the DNA fragment derived from the other member. In this case, the hybridization conditions can be suitable for hybridization with only one allele by facilitating a significant difference in intensities of hybridization for different alleles. According to an embodiment of the present invention, the probe is arranged such that its central site is the polymorphic site of the sequence, for example the 7^{th} position in a probe consisting of 15 nucleotides, or the 8^{th} or 9^{th} position in a probe consisting of 16 nucleotides. In this way, a difference in hybridization for different alleles can be obtained. According to an embodiment of the present invention, the probe can be used in a diagnosis method for detecting an allele, etc. The diagnosis method may be Southern blotting in which detection is performed using the hybridization of nucleic acids, or a method in which a microarray to which the probe is bound in advance is used.

A polypeptide according to an aspect of the present invention is encoded by a polynucleotide of SEQ ID NOS: 1 to 60 and 241 to 244.

Particularly, the amino acid of the polypeptide encoded by the polynucleotide of SEQ ID NO: 56 including MI_1503 located in the CYBA gene changed. The amino acid of the polypeptide encoded by the polynucleotide of SEQ ID NO: 50 including MI_1264 located in the MST1R gene and the polynucleotide of SEQ ID NO: 58 including MI_1248 located in the polymerase iota gene also changed. The sites at which the three amino acids changed are respectively the 72^{nd} amino acid of the CYBA protein, the 1335^{th} amino acid of the MST1R protein and the 706^{th} amino acid of the polymerase iota protein.

An antibody according to an aspect of the present invention specifically binds to the polypeptide. The antibody may be a monoclonal antibody.

A microarray according to an aspect of the present invention includes a polynucleotide of a nucleotide sequence of SEQ ID NOS: 1 to 60 and 241 to 244 and complementary polynucleotides thereof, a polynucleotide hybridized with one of the polynucleotides, a polypeptide encoded by one of the polynucleotides, or cDNA thereof.

The microarray may be prepared using a conventional method known to those skilled in the art using the polynucleotide, a probe or a polynucleotide hybridized with the probe, the polypeptide encoded by one of the polynucleotides or cDNA thereof.

For example, the polynucleotide may be fixed to a substrate coated with an active group of amino-silane, poly-L-lysine or aldehyde. Also, the substrate may be composed of silicon, glass, quartz, metal or plastic. A polynucleotide may be fixed to the subsrate by micropipetting using a piezoelectric method or by using a spotter in the shape of a pin.

A kit according to an aspect of the present invention includes a polynucleotide of a nucleotide sequence of SEQ ID NOS: 1 to 60 and 241 to 244, a polynucleotide hybridized with one of the polynucleotides, a polypeptide encoded by one of the polynucleotides or cDNA thereof.

The kit may further include a primer set used for isolating DNA including SNPs from diagnosed subjects and amplifying the DNA. The appropriate primer set may be determined by those skilled in the art. For example, the primer set in Examples of the present invention may be used. Also, the kit may further include a reagent for a polymerizing reaction, for example dNTP, various polymerases and colorants.

An identifying method according to an aspect of the present invention includes using the SNP to identify a subject having a changed risk of incidence of myocardial infarction.

The identifying method includes isolating a nucleic acid sample from asubject and determining an allele at polymorphic sites of one or more polynucleotides among SEQ ID NOS: 1 to 60 and 241 to 244, wherein the polymorphic sites are positioned at the 101^{st} nucleotides of the polynucleotides.

In an embodiment of the present invention, the isolation of the DNA from the subject can be carried out by performing a method known to those skilled in the art. For example, DNA can be directly purified from tissues or cells or a specific region can be amplified using a polymerase chain reaction (PCR), etc. and isolated. In the detailed description, DNA refers not only to DNA, but also to cDNA synthesized from mRNA. Nucleic acids can be obtained from a subject using PCR amplification, ligase chain reaction (LCR) (Wu and Wallace, Genomics 4, 560(1989), Landegren, etc., Science 241, 1077(1988)), transcription amplification (Kwoh, etc., Proc. Natl. Acad. Sci. USA 86, 1173(1989)), self-sustained sequence replication (Guatelli, etc., Proc. Natl. Acad. Sci. USA 87, 1874(1990)) or Nucleic Acid Sequence Based Amplification (NASBA).

Sequencing of the isolated DNA may be performed through various methods known to those skilled in the art. For example, the nucleotides of nucleic acids may be directly sequenced using a dideoxy method. Also, the nucleotides of the polymorphic sites may be sequenced by hybridizing the DNA with a probe containing the sequence of the SNP site and a complementary probe thereof, and examining the degree of the hybridization. The degree of hybridization may be measured using a method of indicating a detectable index of the target DNA and specifically detecting the hybridized target, or using an electrical signal detecting method.

Particularly, the sequencing may be carried out using allele-specific probe hybridization, allele-specific amplification, sequencing, 5' nuclease digestion, molecular beacon assay, oligonucleotide ligation assay, size analysis or single-stranded conformation polymorphism method.

In an embodiment of the present invention, the method of diagnosing myocardial infarction may further include judging that the subject has an increased risk of incidence of myocardial infarction when an allele at a polymorphic site of one or more polynucleotides of SEQ ID NOS: 1 to 60 and 241 to 244 is a risk allele.

According to an embodiment of the present invention, the risk allele is determined based on the allele 'A'. When a frequency of the allele 'A' in the disease group is higher than in the normalgroup, 'A' is regarded as a risk allele. In the opposite case, 'a' is regarded as a risk allele. subjects having more risk alleles have a higher probability of having myocardial infarction.

In an embodiment of the present invention, the risk may be an increased risk or a decreased risk. When the frequency of an allele is higher in the normal group than in the disease group, the risk to a subject with the allele may be decreased. On the other hand, when the frequency of an allele of the SNP is higher in the disease group than in the normal group, the risk to a subject with the allele can be increased.

In a method of diagnosis of myocardial infarction according to an embodiment of the present invention, the subject may be male and does not smoke and the polynucleotide determining the allele at the polymorphic site in the subject may be a nucleotide sequence of SEQ ID NOS: 57 to 60.

Also, the subject may have a high CRP level and the polynucleotide determining the allele at the polymorphic site in the subject may be a nucleotide sequence of SEQ ID NO: 241, 243 or 244.

The subject may be young and the polynucleotide determining the allele at the polymorphic site in the subject may be a nucleotide sequence of SEQ ID NO: 241, 243 or 244.

The subject may not have diabetes and the polynucleotide determining the allele at the polymorphic site in the subject may be a nucleotide sequence of SEQ ID NO: 241, 243 or 244.

The subject may smoke and the polynucleotide determining the allele at the polymorphic site in the subject may be a nucleotide sequence of SEQ ID NO: 241, 242 or 244.

The subject may have a high TG level and the polynucleotide determining the allele at the polymorphic site in the subject may be SEQ ID NO: 243.

In a method of diagnosing myocardial infarction according to an embodiment of the present invention, a haplotype may be used. The polynucleotide may consist of SEQ ID NOS: 57 to 60. Alternatively, the polynucleotide may consist of SEQ ID NOS: 241 to 244.

A method of detecting SNPs in nucleic acid molecules according to an aspect of the present invention includes contacting a test sample containing nucleic acid molecules with a reagent specifically hybridized under strict conditions with a polynucleotide of a nucleotide sequence of SEQ ID NOS: 1 to 60 and 241 to 244 containing at least 8 contiguous nucleotides and the 101^{st} base of the nucleotide sequence and complementary polynucleotides of the nucleotide sequences, and detecting the formation of a hybridized double-strand.

The detecting of the formation of a hybridized double-strand is carried out using allele-specific probe hybridization, allele-specific amplification, sequencing, 5' nuclease digestion, molecular beacon assay, oligonucleotide ligation assay, size analysis or single-stranded conformation polymorphism.

A method of screening pharmaceutical compositions for myocardial infarction according to an aspect of the present invention includes contacting a candidate material with a polypeptide encoded by a polynucleotide of a nucleotide sequence of SEQ ID NOS: 1 to 60 and 241 to 244 containing at least 8 contiguous nucleotides and the 101^{st} base of the nucleotide sequence and complementary polynucleotides of the nucleotide sequences under proper conditions for the formation of a binding complex, and detecting the formation of the binding complex from the polypeptide and the candidate material.

The detecting the formation of the binding complex may be carried out through coimmunoprecipitation, Radioimmunoassay (RIA), Enzyme Linked ImmunoSorbent Assay (ELISA), Immunohistochemistry, Western Blotting or Fluorescence Activated Cell Sorer (FACS).

A method of regulating gene expression according to an embodiment of the present invention includes binding an anti-sense nucleotide or Si RNA with a polynucleotide of nucleotide sequence of SEQ ID NOS: 1 to 60 and 241 to 244 containing at least 8 contiguous nucleotides and the 101^{st} base of the nucleotide sequence and complementary polynucleotides of the nucleotide sequences, wherein the anti-sense nucleotide and Si RNA are specific to the polynucleotide.

### Advantageous Effects

The SNP and haplotype associated with myocardial infarction according to the present invention may be used for diagnosis and treatment of myocardial infarction and gene fingerprint analysis. By using the microarray and the kit including the SNP of the present invention, myocardial infarction can be effectively diagnosed. According to the method of analyzing SNPs associated with myocardial infarction of the present invention, the presence or risk of myocardial infarction can effectively be diagnosed.

### Best Mode

The present invention will now be described in greater detail with reference to the following examples. The following examples are for illustrative purposes only and are not intended to limit the scope of the invention.

**Example 1**

**SNP selection**

DNA was isolated from blood of a disease group diagnosed with a cardiovascular disease and treated, and DNA was isolated from a normal group not having symptoms of cardiovascular disease, and then an appearance frequency of a specific SNP was analyzed. Both groups consisted of Koreans. The SNP of the Examples of the present invention was selected from either a published database (NCBI dbSNP:http://www.ncbi.nlm.nih.gov/SNP/) or a Sequenom website (http://www.realsnp.com/). The SNPs were analyzed using a primer close to the selected SNP.

1-1. Preparation of DNA sample

DNA was extracted from blood of a disease group consisting of 221 Korean male patients diagnosed with myocardial infarction and DNA was extracted from a normal group consisting of 192 Korean men not having myocardial infarction symptoms. The chromosome DNA extraction was carried out according to a known method (Molecular cloning: A Laboratory Manual, p 392, Sambrook, Fritsch and Maniatis, 2nd edition, Cold Spring Harbor Press, 1989) and the guidelines of a commercially available kit (Gentra system, D-50K). Only DNA having a UV absorvance ratio (260/280 nm) of at least 1.7 was selected from the extracted DNA and used.

1-2. Amplification of the target DNA

Target DNA containing a certain DNA region including SNPs to be analyzed was amplified using a PCR. The PCR was performed using a conventional method and the conditions were as indicated below. 2.5 ng/ml of target genome DNA was first prepared. Then the following PCR reaction solution was prepared.

Water (HPLC grade) 3.14 □□

10 × buffer 0.5 □

MgCl₂ 25 mM 0.2 0

dNTP mix (GIBCO)(25 mM/each) 0.04 □

Taq pol (HocStart)(5 U/ □ ) 0.02 □

Forward/reverse primer mix (10 µM) 0.1 □

DNA 1.00 □

Total volume 5.00 □

Here, the forward and reverse primers were selected upstream and downstream from the SNP at proper positions. The primer set is listed in Table 13.

Thermal cycling of a PCR was performed by maintaining the temperature at 95°C for 15 minutes, cycling the temperature from 95°C for 30 seconds, to 56°C for 30 seconds, to 72°C for 1 minute a total of 45 times, maintaining the temperature at 72°C for 3 minutes, and then storing at 4°C. As a result, target DNA fragments having 200 nucleotides or less were obtained.

1-3. Analysis of SNP of the amplified target DNA

Analysis of the SNPs in the target DNA fragments was performed using a homogeneous Mass Extend (hME) technique from Sequenom. The principle of the hME technique is as follows. First, a primer, also called an extension primer, complementary to bases up to just before the SNP of the target DNA fragment was prepared. Next, the primer was hybridized with the target DNA fragment and DNA polymerization was facilitated. At this time, a reagent (Termination mix, e.g. ddTTP) for terminating the polymerization was added to the reaction solution after the base complementary was added to a first allele base (e.g. 'A' allele) among the subject SNP alleles. As a result, when the target DNA fragment included the first allele (e.g. 'A' allele), a product having only one base complementary to the added first allele (e.g. 'T') was obtained. On the other hand, whe the target DNA fragment included a second allele (e.g. 'G' allele), a product having a base complementary to the second allele (e.g. 'C') and extending to the first allele base (e.g. 'A') was obtained. The length of the product extending from the primer was determined using mass analysis to determine the type of allele in the target DNA. Specific experimental conditions were as follows.

First, free dNTPs were removed from the PCR product. To this end, 1.53 □ of pure water, 0.17 □ of a hME buffer and 0.30 □ of shrimp alkaline phosphatase (SAP) were added to a 1.5 ml tube and mixed to prepare a SAP enzyme solution. The tube was centrifuged at 5,000 rpm for 10 seconds. Then, the PCR product was put into the SAP solution tube, sealed, maintained at 37°C for 20 minutes and at 85°C for 5 minutes, and then stored at 4°C.

Next, homogeneous extension was performed using the target DNA product as a template. The reaction solution was as follows.

Water (nanopure grade) 1.728 □

hME extension mix (10 × buffer containing 2.25 mM d/ddNTPs) 0.200 □

Extension primer (each 100 µM) 0.054 □

Thermosequenase (32 U/ □) 0.018 □

Total volume 2.00 □

The reaction solution was mixed well and spin down centrifuged. A tube or plate containing the reaction solution was sealed, maintained at 94°C for 2 minutes, cycled from 94°C for 5 seconds, to 52°C for 5 seconds, to 72°C for 5 seconds a total of 40 times, and then stored at 4°C. The obtained homogeneous extension product was washed with resins to reduce the amount of salts (SpectroCLEAN, Sequenom, #10053) . The extension primers used for homogeneous extension are indicated in Table 13.

**Table 13**

| Nucleotide containing SNP (SEQ ID NO:) | Primer for target DNA amplification (SEQ ID NO:) | | Extension primer (SEQ ID NO:) |
|---|---|---|---|
| | Forward primer | Reverse primer | |
| 1 | 61 | 62 | 63 |
| 2 | 64 | 65 | 66 |
| 3 | 67 | 68 | 69 |
| 4 | 70 | 71 | 72 |
| 5 | 73 | 74 | 75 |
| 6 | 76 | 77 | 78 |
| 7 | 79 | 80 | 81 |
| 8 | 82 | 83 | 84 |
| 9 | 85 | 86 | 87 |
| 10 | 88 | 89 | 90 |
| 11 | 91 | 92 | 93 |
| 12 | 94 | 95 | 96 |
| 13 | 97 | 98 | 99 |
| 14 | 100 | 101 | 102 |
| 15 | 103 | 104 | 105 |
| 16 | 106 | 107 | 108 |
| 17 | 109 | 110 | 111 |
| 18 | 112 | 113 | 114 |
| 19 | 115 | 116 | 117 |
| 20 | 118 | 119 | 120 |
| 21 | 121 | 122 | 123 |
| 22 | 124 | 125 | 126 |
| 23 | 127 | 128 | 129 |
| 24 | 130 | 131 | 132 |
| 25 | 133 | 134 | 135 |
| 26 | 136 | 137 | 138 |
| 27 | 139 | 140 | 141 |
| 28 | 142 | 143 | 144 |
| 29 | 145 | 146 | 147 |
| 30 | 148 | 149 | 150 |
| 31 | 151 | 152 | 153 |
| 32 | 154 | 155 | 156 |
| 33 | 157 | 158 | 159 |
| 34 | 160 | 161 | 162 |
| 35 | 163 | 164 | 165 |
| 36 | 166 | 167 | 168 |
| 37 | 169 | 170 | 171 |
| 38 | 172 | 173 | 174 |
| 39 | 175 | 176 | 177 |
| 40 | 178 | 179 | 180 |
| 41 | 181 | 182 | 183 |
| 42 | 184 | 185 | 186 |
| 43 | 187 | 188 | 189 |
| 44 | 190 | 191 | 192 |
| 45 | 193 | 194 | 195 |
| 46 | 196 | 197 | 198 |
| 47 | 199 | 200 | 201 |
| 48 | 202 | 203 | 204 |
| 49 | 205 | 206 | 207 |
| 50 | 208 | 209 | 210 |
| 51 | 211 | 212 | 213 |
| 52 | 214 | 215 | 216 |
| 53 | 217 | 218 | 219 |
| 54 | 220 | 221 | 222 |
| 55 | 223 | 224 | 225 |
| 56 | 226 | 227 | 228 |
| 57 | 229 | 230 | 231 |
| 58 | 232 | 233 | 234 |
| 59 | 235 | 236 | 237 |
| 60 | 238 | 239 | 240 |
| 241 | 245 | 246 | 247 |
| 242 | 248 | 249 | 250 |
| 243 | 251 | 252 | 253 |
| 244 | 254 | 255 | 256 |

Mass analysis was performed on the obtained extension product to determine the sequence of a polymorphic site using Matrix Assisted Laser Desorption and Ionization-Time of Flight (MALDI-TOF). In the MALDI-TOF, a material to be analyzed was exposed to laser beam, and flew with an ionized matrix (e.g. 3-Hydroxypicolinic acid) in a vacuum to a detector. The flying time to the detector was calculated to determine the mass. A light material could reach the detector in a shorter amount of time than a heavy material. The nucleotide sequences of SNPs in the target DNA may be determined based on differences in mass and known nucleotide sequences of the SNPs.

1-4. Selection of SNP

Allele frequencies in the disease group consisting of 221 Korean male patients diagnosed with myocardial infarction and treated and allele frequencies of the normal group consisting of 192 Korean men not having symptoms of myocardial infarction were compared. The Fisher's exact test was performed based on the frequency as an association test.

The effect size was assumed using an allele odds ratio at a 95% confidence interval. When the normal group had a higher frequency of a given allele than the disease group, the allele was determined to be associated with a decreased risk of myocardial infarction and the other allele was determined to be the risk allele of myocardial infarction. On the other hand, when the disease group had a higher frequency of a given allele than the normal group, the allele was determined to be the risk allele.

In the allele association test of a SNP, when the p-value was 0.05 or less, the SNP was regarded as a noticeable genetic marker.

The results are listed in Tables 1 to 3. As indicated in Tables 1 to 3, 64 SNPs associated with myocardial infarction were identified.

1-5. Selection of haplotype

Four SNPs of SEQ ID NOS: 57 to 69 in a polymerase iota gene which is associated with DNA repair are disclosed in Table 5. In addition, four SNPs of SEQ ID NOS: 241 to 244 are disclosed in Table 6.

1-6. Investigation into environmental or habitual factor dependence of SNP

The disease group consisting of 221 Korean male patients diagnosed with myocardial infarction and treated and the normal group consisting of 192 Korean men not having symptoms of myocardial infarction were respectively divided into subgroups in consideration of a degree of risk in connection with environmental or habitual factors associated with myocardial infarction, and allele frequencies were compared. The results were shown in Tables 7 to 12.

**Example 2**

**Preparation of SNP immobilized microarray**

A microarray was prepared by immobilizing the selected SNPs on a substrate. That is, polynucleotides of nucleotide sequences of SEQ ID NOS: 1 to 60 and 241 to 244 including 20 contiguous nucleotides and were immobilized on the substrate, wherein each SNP (101^{st} base of the nucleotide sequence) was located at the 11^{th} of the 20 nucleotides.

First, N-terminal ends of each of the polynucleotides were substituted with an amine group and the polynucleotides were spotted onto a silylated slide (Telechem) where 2 × SSC (pH 7.0) of a spotting buffer was used. After spotting, binding was induced in a drying machine and free oligonucleotides were removed by washing with a 0.2% SDS solution for 2 minutes and with triple distilled water for 2 minutes. The microarray was prepared using denaturation induced by increasing the temperature of the slide to 95 °C for 2 minutes, washing with a blocking solution (1.0g NaBH₄, PBS(pH 7.4) 300mL, EtOH 100mL) for 15 minutes, a 0.2% SDS solution for 1 minute and triple distilled water for 2 minutes, and then drying at room temperature.

**Example 3**

**Diagnosis of myocardiar infarction using the microarray**

A target DNA was isolated from blood of a subject to diagnose the incidence or possibility of myocardial infarction and was labeled with a fluorescent material using the methods described in Examples 1-1 and 1-2. The fluorescent labeled target DNA was hybridized with the microarray prepared in Example 2 at 42 °C for 4 hours in a UniHyb hybridization solution (TeleChem). The slide was washed twice with 2 × SSC at room temperature for 5 minutes and dried in air. The dried slide was scanned using a ScanArray 5000 (GSI Lumonics). The scanned results were analyzed using a QuantArray (GSI Lumonics) and an ImaGene software (BioDiscover). The probability of incidence of myocardial infarction and the susceptibility thereto were measured by identifying whether the subject had the SNP according to an embodiment of the present invention.

While the present invention has been particularly shown and described with reference to exemplary embodiments thereof, it will be understood by those of ordinary skill in the art that various changes in form and details may be made therein without departing from the spirit and scope of the present invention as defined by the following claims.
## Claims

1. A polynucleotide for use in the in vitro diagnosis of an increased risk of incidence of myocardial infarction in a subject, wherein the polynucleotide is (i) a polynucleotide selected from the group consisting of nucleotide sequences of SEQ ID NOS: 57 to 60 including at least 8 contiguous nucleotides and the 101^{st} base of the nucleotide sequence and complementary polynucleotides of the nucleotide sequences, or (ii) a polynucleotide specifically hybridized with the polynucleotide of item (i).

2. The polynucleotide for use of claim 1, wherein the polynucleotide consists of a nucleotide sequence selected from SEQ ID NOS: 57 to 60.

3. The polynucleotide for use of claim 1, wherein the subject is male and does not smoke.

4. The polynucleotide for use of claim 1, wherein the polynucleotide comprises 8 to 70 contiguous nucleotides.

5. A primer for use in the in vitro diagnosis of myocardial infarction, said primer specifically hybridizing with a polynucleotide selected from the group consisting of nucleotide sequences of SEQ ID NOS: 57 to 60 including at least 8 contiguous nucleotides and the 101^{st} base of the nucleotide sequence and complementary polynucleotides of the nucleotide sequences, , wherein the primer comprises a length of 15-30 nucleotides, and the 3' end of the primer corresponds to the 101^{st} base of the nucleotide sequence of SEQ ID NOS: 57 to 60, which 101^{st} base is A in SEQ ID NO: 57, is C in SEQ ID NO: 58, is T in SEQ ID NO: 59 and/or is T in SEQ ID NO: 60.

6. A method of identifying a subject having an increased risk of incidence of myocardial infarction, the method comprising: a nucleic acid sample obtained from the subject; determining an allele at a polymorphic site of one or more polynucleotides selected from the group consisting of nucleotide sequences of SEQ ID NOS: 57 to 60, wherein the polymorphic site is positioned at the 101^{st} nucleotide of the polynucleotides; and judging that the subject has an increased risk of incidence of myocardial infarction when the allele at a polymorphic site of one or more polynucleotides selected from the group consisting of nucleotide sequences of SEQ ID NOS: 57 to 60 is a risk allele, wherein the allele is a risk allele if the base at the polymorphic site in SEQ ID NO. 57 is A, in SEQ ID NO. 58 is C, in SEQ ID NO. 59 is T, and/or in SEQ ID NO. 60 is T.

7. The method of claim 6, wherein the determining the allele is carried out by performing a method selected from the group consisting of allele-specific probe hybridization, allele- specific amplification, sequencing, 5' nuclease digestion, molecular beacon assay, oligonucleotide ligation assay, size analysis and single-stranded conformation polymorphism.

8. The method of claim 6, wherein the subject is male and does not smoke and the polynucleotide is selected from the group consisting of nucleotide sequences of SEQ ID NOS: 57 to 60.

9. The method of claim 6, wherein the polynucleotide is consisting of nucleotide sequences of SEQ ID NOS: 57 to 60.

10. A method for use in identifying an increased risk of incidence of myocardial infarction, comprising detecting an SNP in nucleic acid molecules, the method comprising: contacting a test sample containing nucleic acid molecules with a reagent specifically hybridized under strict conditions with a polynucleotide selected from the group consisting of nucleotide sequences of SEQ ID NOS: 57 to 60 comprising at least 8 contiguous nucleotides and the 101^{st} base of the nucleotide sequence and complementary polynucleotides of the nucleotide sequences; and detecting the formation of a hybridized double-strand, wherein the 101^{st} base of the nucleotide sequence in SEQ ID NO. 57 is A, in SEQ ID NO. 58 is C, in SEQ ID NO. 59 is T, and/or in SEQ ID NO. 60 is T.

11. The method of claim 10, wherein the detecting the formation of a hybridized double-strand is carried out by performing a method selected form the group consisting of allele-specific probe hybridization, allele- specific amplification, sequencing, 5' nuclease digestion, molecular beacon assay, oligonucleotide ligation assay, size analysis and single-stranded conformation polymorphism.

## Patentansprüche

1. Polynucleotid zur Verwendung bei der in-vitro-Diagnose eines erhöhten Risikos für das Auftreten eines Myokardinfarktes bei einem Probanden, wobei es sich bei dem Polynucleotid (i) um ein Polynucleotid, ausgewählt aus der Gruppe, bestehend aus Nucleotidsequenzen der SEQ ID NO: 57 bis 60, die mindestens 8 zusammenhängende Nucleotide und die 101. Base der Nucleotidsequenz einschließen, und komplementären Polynucleotiden der Nucleotidsequenzen, oder (ii) um ein spezifisch mit dem Polynucleotid nach Punkt (i) hybridisiertes Polynucleotid handelt.

2. Polynucleotid zur Verwendung nach Anspruch 1, wobei das Polynucleotid aus einer Nucleotidsequenz, ausgewählt aus SEQ ID NO: 57 bis 60, besteht.

3. Polynucleotid zur Verwendung nach Anspruch 1, wobei der Proband männlich ist und nicht raucht.

4. Polynucleotid zur Verwendung nach Anspruch 1, wobei das Polynucleotid 8 bis 70 zusammenhängende Nucleotide umfasst.

5. Primer zur Verwendung bei der in-vitro-Diagnose eines Myokardinfarktes, wobei der Primer spezifisch mit einem Polynucleotid hybridisiert, ausgewählt aus der Gruppe, bestehend aus Nucleotidsequenzen der SEQ ID NO: 57 bis 60, die mindestens 8 zusammenhängende Nucleotide und die 101. Base der Nucleotidsequenz einschließen, und komplementären Polynucleotiden der Nucleotidsequenzen, wobei der Primer eine Länge von 15 - 30 Nucleotiden umfasst und das 3'-Ende des Primers der 101. Base der Nucleotidsequenz der SEQ ID NO: 57 bis 60 entspricht, wobei es sich bei der 101. Base in SEQ ID NO: 57 um A, in SEQ ID NO: 58 um C, in SEQ ID NO: 59 um T und/oder in SEQ ID NO: 60 um T handelt.

6. Verfahren zum Identifizieren eines Probanden mit einem erhöhten Risiko für das Auftreten eines Myokardinfarktes, wobei das Verfahren umfasst: eine von dem Probanden erhaltene Nucleinsäureprobe; Bestimmen eines Allels an einer polymorphen Stelle eines oder mehrerer Polynucleotide, ausgewählt aus der Gruppe, bestehend aus Nucleotidseguenzen der SEQ ID NO: 57 bis 60, wobei sich die polymorphe Stelle am 101. Nucleotid der Polynucleotide befindet; und Beurteilen, dass der Proband ein erhöhtes Risiko für das Auftreten eines Myokardinfarktes aufweist, wenn es sich bei dem Allel an einer polymorphen Stelle eines oder mehrerer Polynucleotide, ausgewählt aus der Gruppe, bestehend aus Nucleotidsequenzen der SEQ ID NO: 57 bis 60, um ein Risikoallel handelt, wobei es sich bei dem Allel um ein Risikoallel handelt, falls es sich bei der Base an der polymorphen Stelle in SEQ ID NO: 57 um A, in SEQ ID NO: 58 um C, in SEQ ID NO: 59 um T und/oder in SEQ ID NO: 60 um T handelt.

7. Verfahren nach Anspruch 6, wobei das Bestimmen des Allels ausgeführt wird, indem ein Verfahren durchgeführt wird, ausgewählt aus der Gruppe, bestehend aus allelspezifischer Sondenhybridisierung, allelspezifischer Amplifikation, Sequenzierung, 5'-Nucleaseverdau, molekularer Beacon-Assay, Oligonucleotid-Ligations-Assay, Größenanalyse und Einzelstrang-Konformationspolymorphismus.

8. Verfahren nach Anspruch 6, wobei der Proband männlich ist und nicht raucht und das Polynucleotid aus der Gruppe ausgewählt ist, bestehend aus Nucleotidsequenzen der SEQ ID NO: 57 bis 60.

9. Verfahren nach Anspruch 6, wobei das Polynucleotid aus Nucleotidsequenzen der SEQ ID NO: 57 bis 60 besteht.

10. Verfahren zur Verwendung beim Identifizieren eines erhöhten Risikos des Auftretens eines Myokardinfarktes, umfassend das Nachweisen eines SNPs in Nucleinsäuremolekülen, wobei das Verfahren umfasst: In-Kontakt-Bringen einer Testprobe, die Nucleinsäuremoleküle enthält, mit einem Reagens, unter strikten Bedingungen spezifisch mit einem Polynucleotid hybridisiert, ausgewählt aus der Gruppe, bestehend aus Nucleotidsequenzen der SEQ ID NO: 57 bis 60, umfassend mindestens 8 zusammenhängende Nucleotide und die 101. Base der Nucleotidsequenz, und komplementären Polynucleotiden der Nucleotidsequenzen; und Nachweisen der Bildung eines hybridisierten Doppelstranges, wobei es sich bei der 101. Base der Nucleotidsequenz in SEQ ID NO: 57 um A, in SEQ ID NO: 58 um C, in SEQ ID NO: 59 um T und/oder in SEQ ID NO: 60 um T handelt.

11. Verfahren nach Anspruch 10, wobei das Nachweisen der Bildung eines hybridisierten Doppelstranges ausgeführt wird, indem ein Verfahren durchgeführt wird, ausgewählt aus der Gruppe, bestehend aus allelspezifischer Sondenhybridisierung, allelspezifischer Amplifikation, Sequenzierung, 5`-Nucleaseverdau, molekularer Beacon-Assay, Oligonucleotid-Ligations-Assay, Größenanalyse und Einzelstrang-Konformationspolymorphismus.

## Revendications

1. Polynucléotide pour utilisation dans le diagnostic in vitro d'un risque accru d'incidence d'infarctus du myocarde chez un sujet, lequel polynucléotide est (i) un polynucléotide choisi dans le groupe constitué par les séquences nucléotidiques des SEQ ID NOS: 57 à 60 incluant au moins 8 nucléotides contigus et la 101^{e} base de la séquence nucléotidique et les polynucléotides complémentaires de ces séquences nucléotidiques, ou (ii) un polynucléotide spécifiquement hybridé au polynucléotide du point (i).

2. Polynucléotide pour utilisation selon la revendication 1, lequel polynucléotide est constitué d'une séquence nucléotidique choisie parmi les SEQ ID NOS: 57 à 60.

3. Polynucléotide pour utilisation selon la revendication 1, où le sujet est de sexe masculin et ne fume pas.

4. Polynucléotide pour utilisation selon la revendication 1, lequel polynucléotide comprend 8 à 70 nucléotides contigus.

5. Amorce pour utilisation dans le diagnostic in vitro de l'infarctus du myocarde, ladite amorce s'hybridant spécifiquement à un polynucléotide choisi dans le groupe constitué par les séquences nucléotidiques des SEQ ID NOS: 57 à 60 incluant au moins 8 nucléotides contigus et la 101^{e} base de la séquence nucléotidique et les polynucléotides complémentaires de ces séquences nucléotidiques, où l'amorce comprend une longueur de 15-30 nucléotides, et l'extrémité 3' de l'amorce correspond à 101^{e} base de la séquence nucléotidique des SEQ ID NOS: 57 à 60, laquelle 101^{e} base est A dans SEQ ID NO: 57, est C dans SEQ ID NO: 58, est T dans SEQ ID NO: 59, et/ou est T dans SEQ ID NO: 60.

6. Méthode pour identifier un sujet présentant un risque accru d'incidence d'infarctus du myocarde, la méthode comprenant: un échantillon d'acide nucléique obtenu auprès du sujet ; la détermination d'un allèle au niveau d'un site polymorphe d'un ou de plusieurs polynucléotides choisis dans le groupe constitué par les séquences nucléotidiques des SEQ ID NOS: 57 à 60, le site polymorphe étant positionné au niveau du 101^{e} nucléotide de ces polynucléotides ; et l'estimation que le sujet présente un risque accru d'incidence d'infarctus du myocarde lorsque l'allèle au niveau d'un site polymorphe d'un ou de plusieurs polynucléotides choisis dans le groupe constitué par les séquences nucléotidiques des SEQ ID NOS: 57 à 60 est un allèle à risque, l'allèle étant un allèle à risque si la base au niveau du site polymorphe dans SEQ ID NO. 57 est A, dans SEQ ID NO. 58 est C, dans SEQ ID NO. 59 est T, et/ou dans SEQ ID NO. 60 est T.

7. Méthode selon la revendication 6, dans laquelle on procède à la détermination de l'allèle en mettant en oeuvre une méthode choisie dans le groupe constitué par l'hybridation de sonde spécifique d'allèle, l'amplification spécifique d'allèle, le séquençage, la digestion par nucléase agissant en 5', la technique des balises moléculaires, la technique de ligature d'oligonucléotides, l'analyse de la taille, et le polymorphisme de conformation simple brin.

8. Méthode selon la revendication 6, dans laquelle le sujet est de sexe masculin et ne fume pas et le polynucléotide est choisi dans le groupe constitué par les séquences nucléotidiques des SEQ ID NOS: 57 à 60.

9. Méthode selon la revendication 6, dans laquelle le polynucléotide est constitué des séquences nucléotidiques des SEQ ID NOS: 57 à 60.

10. Méthode pour utilisation dans l'identification d'un risque accru d'incidence d'infarctus du myocarde, comprenant la détection d'un SNP dans des molécules d'acide nucléique, la méthode comprenant: la mise en contact d'un échantillon d'essai contenant des molécules d'acide nucléique avec un réactif spécifiquement hybridé dans des conditions strictes à un polynucléotide choisi dans le groupe constitué par les séquences nucléotidiques des SEQ ID NOS: 57 à 60 comprenant au moins 8 nucléotides contigus et la 101^{e} base de la séquence nucléotidique et les polynucléotides complémentaires de ces séquences nucléotidiques ; et la détection de la formation d'un double brin hybridé, où la 101^{e} base de la séquence nucléotidique dans SEQ ID NO. 57 est A, dans SEQ ID NO. 58 est C, dans SEQ ID NO. 59 est T, et/ou dans SEQ ID NO. 60 est T.

11. Méthode selon la revendication 10, dans laquelle on procède à la détection de la formation d'un double brin hybridé en mettant en oeuvre une méthode choisie dans le groupe constitué par l'hybridation de sonde spécifique d'allèle, l'amplification spécifique d'allèle, le séquençage, la digestion par nucléase agissant en 5', la technique des balises moléculaires, la technique de ligature d'oligonucléotides, l'analyse de la taille, et le polymorphisme de conformation simple brin.


<110> Samsung Electronics Co., Ltd
<120> Genetic polymorphisms associated with myocardial infarction and uses thereof
<130> PX024788
<160> 256
<170> KopatentIn 1.71
<210> 1
   <211> 200
   <212> DNA
   <213> Homo sapiens
<400> 1
   aagaatggaa ggacagcttg ctacttctgg gtgttcatca tgagcagtta aggctcacca      60
   tatggtctcc actgatattt gggggaagga gacttgttac yacctaataa tgagatgaaa     120
   gatcctgatt ccctacttgg tttactatga caccgccctg gcatgatggt tcaggaaact     180
   ctttacattt acagattgtc                                                 200
<210> 2
   <211> 200
   <212> DNA
   <213> Homo sapiens
<400> 2
   tactgctgtc tttggaacac tgttactcat cctacaaggc ctcaatcaag aatcttagga      60
   ggtcattggt gcctacctgc ccatggcaga gaccaccaac mccttctgag agctgccaca     120
   gtgctgcttt ctaacatgtc tacactagct cctcacagtg tgcggtgatt gttgggtgaa     180
   agctctgttt ctcatagtga                                                 200
<210> 3
   <211> 200
   <212> DNA
   <213> Homo sapiens
<400> 3
   tgatttgatt ttatccagtg ttgcaaactg ggtttcgtaa gtgactcttt tgtaaggagt      60
   ttcagtgcac cccgtctgca acccatgcat atcttattta mccccaagct tctaaggtag     120
   aatcctactt ttttcaagtg ctattgatta gttgcctgtt tagacttttg ttatatgatt     180
   taaaatttta aaaatgataa                                                 200
<210> 4
   <211> 200
   <212> DNA
   <213> Homo sapiens
<400> 4
   gtgtttctaa agcaactccc caccaacctc ttgggctgtg gtgaccaagg gctaaaaatt      60
   aagtggggac cctggtgtct gacttttgct ccacttctga yaccttcttt ctctcccgtt     120
   tcactaggac tggaaaatac aggctttttt cccaatcctt gtttctctca aaccaagacc     180
   tcatctacat aaaacatcta                                                 200
<210> 5
   <211> 200
   <212> DNA
   <213> Homo sapiens
<400> 5
   ccactgaaaa tatcctctgg tcatttattt ggctcaacgg acctatatgg ccattccctc      60
   ttccacagtt gtaaagactt tgcctaattt ctggcaacta yagaagtagt ggtaaggata     120
   atgtaaaatt tctttttgtc ttggaaatta tcaaaccact tttgaagatt gtattatcta     180
   gtaatcagaa acttactttg                                                 200
<210> 6
   <211> 166
   <212> DNA
   <213> Homo sapiens
<400> 6
   taagggcttg agtatgcaaa tatggaggct ttctcaaatc acgtttcgtt taatttaaca      60
   ccttcataaa taggatggac ttaagagatc atcccaacct ycttactgta cagatcaaga     120
   gatgggggtc cggaaagctg ctgatgttca tgctgtgagt ttaaca                    166
<210> 7
   <211> 200
   <212> DNA
   <213> Homo sapiens
<400> 7
   tttgccaacc attcttcacc ctgttgggag agaatacaga aatctgtatt caagcataag      60
   gtgctataca cttgtttgat gcctgcctct agggcaatga rgaggtaaga caatctcccc     120
   attacatgct ttcgcagagg acagtctgta aagttacaga actccattcc ttcttacgta     180
   ccttatacca taatatacca                                                 200
<210> 8
   <211> 152
   <212> DNA
   <213> Homo sapiens
<400> 8
   aaggggcggc gctggccgga ctctctcggg tggactcccg gagcggcggc ggcggcggcg 60
   gtggcagact gcgagtcacg tgcgacagag gaggcggagc rcggcggccg ggtgggtgga 120
   gaaaacaaag cccccagctg tcagcagagg aa 152
<210> 9
   <211> 200
   <212> DNA
   <213> Homo sapiens
<400> 9
   attatataaa tgaaactggt ttaggatgtg tctttttttc ccctctcttt taccttaact 60
   caggttcaag cctttcttaa gctctggtaa cttgcatata yaacaaagtt gaaataaaac 120
   agatttttat atagggaaag agagatgagg aagggaacat atccagtttt ctcactgtga 180
   aacagagtta gcaaggaagt 200
<210> 10
   <211> 201
   <212> DNA
   <213> Homo sapiens
<400> 10
   gattatgtta acagaaattc tcagcctgtg tgatttgtca tattttaata tttcaagcca 60
   ccattgcagc ttcttttagg tttggtttgt ctgaggcacc rcagcgagtg catgagcaca 120
   cagactttga atttgtataa gtgaatggct cttccactta ctttgtggct ttgggctcat 180
   cacttaactc attgatataa a 201
<210> 11
   <211> 200
   <212> DNA
   <213> Homo sapiens
<400> 11
   tgtgcagcaa tagtaactgg aacaacattt ctgcgatggg cagaagtggc aaggattgta 60
   actcattaga ctcagaaggc gaggtcacct gagtaagcag kgtccttagt ggaacagtgg 120
   gtgtaagagg aaggagagat gggtgggtgt ggggcttctt gccatctaca tatgtagggt 180
   ctgcctttta cttgcacatc 200
<210> 12
   <211> 166
   <212> DNA
   <213> Homo sapiens
<400> 12
   gatgatactt agcttctgcg tcttcagtaa actcatctgt acaatggcct gttaaaccta 60
   gctatttgaa aacatcagct aaaactaagt gatttttttg rtattcattt aggtctttta 120
   tttcttgata tgcttgaaat ctaattgtct tttaaatgca ctaata 166
<210> 13
   <211> 151
   <212> DNA
   <213> Homo sapiens
<400> 13
   gattacaggc gtgagccacg gcacctggag agaaaaaagc ttctttaatg acgacctcaa 60
   cataatttac atggagaagt ttcttaggtg gaaaaccgat ytgtatggtc ctcagaatgt 120
   aacctacagg ttcttgccct cagaaaaatg c 151
<210> 14
   <211> 200
   <212> DNA
   <213> Homo sapiens
<400> 14
   taaggaagag ggacagctat gacctaatgc ttgcttggac cactataagc atgccaggga 60
   aaatattcag gctaaattgt cggctctaag aacatgaagt rtattgattt ctttattaca 120
   gctagcagat atttaagaat gttagcacag gtctttgaat aaattttgct tttaagagaa 180
   gttactattt attcctaatt 200
<210> 15
   <211> 200
   <212> DNA
   <213> Homo sapiens
<400> 15
   tggacaatgg gaataaagca tttctaaagc accgactgga gaggaaggca acagagacaa 60
   ggagagaagc cgagagacat gtctgcgtgc tgccacgcat ytgagcgatt gctctgtgaa 120
   gagttgtaca ctgaacactt tcaggggagg ctgtttaccc aggcaatgtc ctcaaacaag 180
   cctgtgccgg ggtgtcctgg 200
<210> 16
   <211> 200
   <212> DNA
   <213> Homo sapiens
<400> 16
   agaggcggcg gctccggaac gagctgctcc cacagcccct ccgctcctcg ccgtctcgcc 60
   ttcgtcccca gcttacccag ttctgcgcgt tattgctcag yttgactttc ttgcacaggc 120
   tcccggggat ctccatgacc gcggagcgcg ctgagcagcc ggggttctct gcgccgggan 180
   ggtagcaaga ggagggcagg 200
<210> 17
   <211> 200
   <212> DNA
   <213> Homo sapiens
<400> 17
   ttagcagtcc cgcaaagctt gaggtttact catttttact ttctttagtc tctgggcaag 60
   gaactaagag gtagtataga attctagacg atggaaactt yagaatggac aggattttaa 120
   aaattatata ttccattctc ctgggttaca ggtggtctta cagctaaact atttgagact 180
   gttgaagcca ttcgattttt 200
<210> 18
   <211> 200
   <212> DNA
   <213> Homo sapiens
<400> 18
   ttatgaggaa cagatgagat agtgaggttg agtgccagca cagtagccag tactcaatca 60
   aatataactg taaaacaaag gggcagttcc taaggcccaa ratggagttt tagtcaaaag 120
   gaaataaggc aaaaggccag ttacaagatg gagcaggccc tgcagtggag ttgaaaaccc 180
   aggtcaccat gatgactgtg 200
<210> 19
   <211> 200
   <212> DNA
   <213> Homo sapiens
<400> 19
   ttaggaaatt ttcaatggag aaaataagtt ccaaaagtca ttctttctca gattaagatg 60
   tcttgggttt cagtttactt ctttaaagaa agattcagag ytagtactga ggacccagac 120
   tttcttgatg tgggaaatag ataattttct gttctgttga cattttttct ctctcttctc 180
   tcattttcaa agtacagttc 200
<210> 20
   <211> 200
   <212> DNA
   <213> Homo sapiens
<400> 20
   taccatcctc acatcttgta tagactcctt ttatcagggc ctggtggtcc cagttcttcc     60
   tagagaaacc catagcaaca ttcttgatgg tcagtggtcc rtggactggg ggggtcccca    120
   tggccatctg tccttcctct tggctccctt ctgaggggca ggcagggtcc taaaaggaca    180
   gagccctgtg gacctggcag                                                200
<210> 21
   <211> 173
   <212> DNA
   <213> Homo sapiens
<400> 21
   aaacattgtt tacataagta aacaagccag ggctgctgtg aaagttatta tggaagatac     60
   aatttatata ttttctggaa aatatatcgc ctcatatata yagacaaaac ctttgcttta    120
   acatgtttgt gcaactaatt acttactttc caatgagtac aatttcccac tag           173
<210> 22
   <211> 200
   <212> DNA
   <213> Homo sapiens
<400> 22
   gaggtgggca ctggtggctt ccagcctagg gctgtgactc tgctgttagg ggcacgctgc     60
   tacacaggca ccaacctctc tagcacaggg gttcctccaa ktgtgctgtg ggggcccccg    120
   agaccctttc aggggttccc ccaggttcct tgaaaggctc cnctgattag atcaggccca    180
   ccaagatcac ctccctttgg                                                200
<210> 23
   <211> 201
   <212> DNA
   <213> Homo sapiens
<400> 23
   agagtcataa gaagggtttt ctctctccta aggaggaacc aaagggggaa aaaaagtctc     60
   tttgcttcta gacgttgttt ctgtagatgt aaaggctgtt macttctgca gtcatcttgc    120
   tgagaataaa ttcagaatga agatggcaaa atggagcaag aaataagtta atgacccgga    180
   aggcctcctt ctgaagttgt t                                              201
<210> 24
   <211> 200
   <212> DNA
   <213> Homo sapiens
<400> 24
   caccatttat ttcctatttt ctctcctgag ttaaatagga aacatgtctc gcattacttg 60
   aaaaatcaag tcaaactatg ctcttactag gagttatggt yctttttatg tcttagatga 120
   tgcttgatct agatgaatgc ggacttgctg tagctagata aatacaatgg gagtttgaag 180
   gtgtttcgta gccctggaaa 200
<210> 25
   <211> 200
   <212> DNA
   <213> Homo sapiens
<400> 25
   agaactgtgg agcgattcct gatttttgag caggaagagt gacaattcaa aacagtattt 60
   gactagattc acggctccgt agcatcccct tgggtgggag sgggaaggct gactaggacc 120
   tctgattctt ctttccctga gctttgaagg ctctgaaaat acagctgggg ggacttgccc 180
   agttttctta ttaagcaatt 200
<210> 26
   <211> 200
   <212> DNA
   <213> Homo sapiens
<400> 26
   agcacagttc caagatgcgc tgacatgttc tgcaggcgag agagcctttc gctggactgt 60
   gactcacgga ggtggtgagg ccgctggttc taaccaagag ratgccagcg agaccactca 120
   gcctcccctg gtggctgccc tggtttaatg ctcatgaaat gccagcctgt ggccattctg 180
   ctgggtaaag agggtggtgt 200
<210> 27
   <211> 125
   <212> DNA
   <213> Homo sapiens
<400> 27
   cccagcagga acccttctgg aagggaatat ttgcagagat ttcccagatc tatgtgccct 60
   tctgcttcct ctatgcttac tgctctcacc tgggccaccc mtgaaggtgg gcttgctgtt 120
   tcagc 125
<210> 28
   <211> 200
   <212> DNA
   <213> Homo sapiens
<400> 28
   tggcgcgtgt gcaattagca atgcagtgta tatacaagaa gccatgctgt taacagttta 60
   tctcaagtaa tttggtgtca tttacaaaag gaagaaattc ytgccatcag aagggacaga 120
   aggagatgga atgatcaaat cacagagaaa cactaaaatt actaaatcta caacagctcg 180
   ccttattttt cttggaccca 200
<210> 29
   <211> 200
   <212> DNA
   <213> Homo sapiens
<400> 29
   gagacaacgg ggatcaggca gaggggtggg cacataggag gcaacgtaaa ccagaactga 60
   ggctgggcca ggccagggtg tcagaggctg aggcggggag kcgggctgcc cttgggctgg 120
   attgcaaggc gaagaagcag gagggagagg ctgagagtgt ggaggggagg gggcttgtag 180
   cgtgtgattg gagggaggac 200
<210> 30
   <211> 200
   <212> DNA
   <213> Homo sapiens
<400> 30
   ctacccacaa aagtttcttt gtacttattt acnagtcctg atgtttttgt tagaaaaaca 60
   ttgccacctn tttttaccca gccaggtctc agcctaagtg ycactgggtc cagtttctct 120
   catcttctga gcagttggcc taaagtgacc ctatgttttc cttttttctt tctcctcttc 180
   ctcttccaca ccagtgcaaa 200
<210> 31
   <211> 200
   <212> DNA
   <213> Homo sapiens
<400> 31
   tttttaggct taaaaagata ttttaatgta actctatcca attctcaatt ttaggcctgc 60
   cagaattgaa actcaagctt aactataatc atcaccaagc rttacttttg aagccctccc 120
   gtaaatatga ggctgttaag gatgcaatac tataccgcgt gtctcttagg ttagttggaa 180
   aaatgagtta gaaaatgagg 200
<210> 32
   <211> 200
   <212> DNA
   <213> Homo sapiens
<400> 32
   aaagctgagt aagcacctat agtaacaggt aacaaagtat tctagggatc agctgctgtg 60
   agttagttca caagtgcttt aagtacgtgt taatcaaaga ygatgtattt ttcatttctg 120
   acaataaaca tctggggcat cctacatttt tcctgggcaa tttgccttta tcattatatt 180
   ttaatctctg atgcaatctt 200
<210> 33
   <211> 200
   <212> DNA
   <213> Homo sapiens
<400> 33
   ccacttccct ctcacacaga atagatttcc atagggtgag aattcaagag aaacttttta 60
   attgtacagc aaagtgttta ctttgccacc aacctgccct rtagcttaag catatggcta 120
   ccttttctat caacagctgg gcttctgtag gtttggcact cggtatacca tcccagcgca 180
   gtcaaaaacc tgcaggtgac 200
<210> 34
   <211> 200
   <212> DNA
   <213> Homo sapiens
<400> 34
   atgttgttca gagggctatt cagagaatgg aaacccaagg ctcacctttc ttgagtctaa 60
   gtacccactc agccaagtat atttagcaaa atagaaaaac yaaactgctt tggtgactgg 120
   actttgccaa tgtctgaaag aaagaagtaa cagaaaggag agtctaaagt tgatcctgta 180
   cagtataaaa catttcccca 200
<210> 35
   <211> 200
   <212> DNA
   <213> Homo sapiens
<400> 35
   tggatgtact catagagagg gctggagcgc actgggacac tggcaaactg atgagtgccg 60
   ggctccagaa gccgcccatg gcccttccct tcttgggatt ktatctcccc gacttggcgg 120
   tgcctctgtt cctccttctg ctggcggcgg atgtgttctt cccgtaattt cctttcccgt 180
   tcctggcgac gtttctgctc 200
<210> 36
   <211> 200
   <212> DNA
   <213> Homo sapiens
<400> 36
   gggaataaat tagttttcct tgacagtata tactgcctgt ttctgtccat cctcttattt 60
   gcttcttttt ctttgctctt ccactccagt ccttaagcga ktttagtttt attcgttcct 120
   cttgtccctt ttcatgataa caatgtgtca tagtctgctt taaagttttt catacttacc 180
   gatgttagtt ttattgctag 200
<210> 37
   <211> 200
   <212> DNA
   <213> Homo sapiens
<400> 37
   tggggacaga gcctgtgggg agtcttgatg tagtcttgtc cttgaaatcc attcacggct 60
   ctgctgagat ctcgggaaag agtctcttta taaatatggg kttttgtttt ttcaaatttt 120
   ggaaaagtca cctctggttt taaaacaatt acttcccgct ccaaacagat tagtcacttc 180
   ccaagaacca tgcttctctc 200
<210> 38
   <211> 200
   <212> DNA
   <213> Homo sapiens
<400> 38
   attatagaca agcagagagg gaatggatcc gagggtgacc tggctgaatt aaaagtactg 60
   catctaagtg tgtgcaggtt gtagtccaca gtaatggtgc yattcggagc tgtgtgtacc 120
   tgcactactg catcaggtga ccttatccag cataacggta caacagaaat ttaacttgaa 180
   acaggtttat tgacttgctt 200
<210> 39
   <211> 201
   <212> DNA
   <213> Homo sapiens
<400> 39
   gtgacaaacc tgagactaga aaactactac atatgacatc ctacacactg actcccttct 60
   tcagctctgt ctgataaagg gatgagagat cgtttcttat yattacacat acacgccact 120
   tccgttcctt ccatctaaac ctagttcaga aacaccgcta agaattccca cccccaaaaa 180
   aatggggatg ggggaagaga a 201
<210> 40
   <211> 200
   <212> DNA
   <213> Homo sapiens
<400> 40
   actgaggtta gtctcttgta ttaaactctt cacaaaatct gtttagcagc agcctttaat 60
   gcatctagat tatggagctt ttttccttaa tccagctgat sagttacagc ctgttagtaa 120
   catgagggga cattttggtg agaaatggga cttaactcct tccagtgtcc ttagaacatt 180
   ttaattcatc ccaactgtct 200
<210> 41
   <211> 200
   <212> DNA
   <213> Homo sapiens
<400> 41
   cgagactcaa gataatgtac taaagacctt tagcacaatg tctgacatct ggtgttcaat 60
   aagtggtgat attgctcaat ttcattccaa ttcaaactta yaaacatcca tgggaagtct 120
   gctttataga caagcaatag gggagcacag taattatttg ggaggggaaa tctcagcagt 180
   tcttccaagt gacacattct 200
<210> 42
   <211> 200
   <212> DNA
   <213> Homo sapiens
<400> 42
   atttgctgac tttgccaagc gatgagccac acaggattgg gaagcctgaa tgtcatcagt     60
   caaggcccag agtgctgaac ctatgaggcc tggggagacc rctgtgaatt cctgcatcgt    120
   cactgagatc atcatatcct gtgaaaaaca tctgccagcc actgtctgtg ttgtagaaag    180
   ctgctgatgg gaaaacttgg                                                200
<210> 43
   <211> 200
   <212> DNA
   <213> Homo sapiens
<400> 43
   cataaaccct caataaacct aagctactat tattaagtac tgtgtgtcca cacaaaaaaa     60
   aggtctggaa agatatagcc accaggagta gttatgagga rtgaggcaga gaggctgatg    120
   gaggacttcc attttctatt  ttgtatacta cttttctagt gtttacaaat gagcttgtat    180
   tgtttttatc atcagcaaaa                                                200
<210> 44
   <211> 200
   <212> DNA
   <213> Homo sapiens
<400> 44
   atagcaggcc agggctctgt aggcttgttt tgctgctgtg cctgttgcca aatgcttgaa     60
   aatcctgaag tacaaacata acagtctgaa ttaaatttca ytgattttat ctttctcttc    120
   cttttctcac tgtggtgcag ttgttaatgg aattctagcc aaaaggatgt gggaggagag    180
   gaaggagcca caaagggaga                                                200
<210> 45
   <211> 200
   <212> DNA
   <213> Homo sapiens
<400> 45
   ttagtcatga tgttctggtt tttatttata cattcatctg aaagagcaaa gtggtagagc     60
   taaaatcagt aagcttggct ctgtttccaa actcataaaa rtgcaatatc gtggcagaaa    120
   gattcttgtc ctatttctta ttttacctgg gcttttggga tttcacatgg acatagttgc    180
   aaaataatac tccccaaact                                                200
<210> 46
   <211> 200
   <212> DNA
   <213> Homo sapiens
<400> 46
   ctaaccaagc ttgcccatat ttcacatgtt gagtttgttc attttaactg tcaattggcc     60
   taattttccc atttctagca ttttaatttc tccagagctt kcatactaca ttgaacacca    120
   attcagtgac ctggaacaat tccactcgca tttgttcctt ttagaccatt tcactgggag    180
   tatgtttcca atccacttat                                                 200
<210> 47
   <211> 200
   <212> DNA
   <213> Homo sapiens
<400> 47
   cagagacctg gtcctaaatc tgtgtgactt ttgcgaaatc cttttgcctc acagagctct     60
   aggttcttat ctgtcaaaca ggaaccagac catcagatcc rtagagatcc aaccaatgaa    120
   ggagacttcg aaaacttcac attgccgtgt caatgtgagg ttgctggatg gttatgacaa    180
   gaaatcacat taaattcact                                                 200
<210> 48
   <211> 200
   <212> DNA
   <213> Homo sapiens
<400> 48
   gcaggaggaa gggtgcctga ttcacagggg agatgcctga tggcgggtgg ggggctgctg     60
   ttggcatccc cactggaacg tttgtcctaa tgggggcctg rctccctcat cagtggcttg    120
   tcccaaaaca acaaccagtg ttcccaaacc tatacttcct ctttggattt ttgtttttca    180
   tctagttgga gggaataaaa                                                 200
<210> 49
   <211> 200
   <212> DNA
   <213> Homo sapiens
<400> 49
   tcattagttt attgcatccc caaggctttg ataacaaatg aagcaaatat tttcaaattt 60
   ttctggttac ccaataagaa aaatcatgtg actttccaca yctgccatca ttggaagtga 120
   caaaactcta gatacttggg tcattgttat catttgtcta tcttccttgg tatgctctat 180
   tcttcgttgt ggagtaataa 200
<210> 50
   <211> 200
   <212> DNA
   <213> Homo sapiens
<400> 50
   ccagggtcgg cgcctgcccc agcctgagta ttgccctgat tctctgtacc aagtgatgca 60
   gcaatgctgg gaggcagacc cagcagtgcg acccaccttc rgagtactag tgggggaggt 120
   ggagcagata gtgtctgcac tgcttgggga ccattatgtg cagctgccag caacctacat 180
   gaacttgggc cccagcacct 200
<210> 51
   <211> 200
   <212> DNA
   <213> Homo sapiens
<400> 51
   tctgacattc tctggttata aaactagagt atcaaaatgc atgagagtgc ctgctcagat 60
   aaccatcaga tgaatatgaa gcttgctcat ccctgggagt rcagaaatca gctcctttta 120
   cctcttgaag aagtggatac agcatcctct actgctcaat tgcataaacc agcacttgtc 180
   cttggtgagc cgttgtttgc 200
<210> 52
   <211> 200
   <212> DNA
   <213> Homo sapiens
<400> 52
   atagaatttt tggtatactg aacaaaggga ttaaaaaggt cagttttatg tagggacttt 60
   ctttgctggg gagggaaggt ctcatcccca agaattattt mtttattttc ccttcccaga 120
   gtttttacat ggaagtatga aatattacct tcctactata taattaatag aaaaaataaa 180
   cagagcccag attatactat 200
<210> 53
   <211> 200
   <212> DNA
   <213> Homo sapiens
<400> 53
   gcatagtact cagactcatt gaaagtttat agaatttggc ctgtgtggaa aactctgtgc      60
   tccaagtaca acaactaact gaattctcta atttaaacaa stttgaaggg aagtgaaagg     120
   ttataaaatg atacagactc ataccgcaga atcaccttaa aatgcagctg ttggagaaaa     180
   aaaaaaagga agctttatgc                                                 200
<210> 54
   <211> 176
   <212> DNA
   <213> Homo sapiens
<400> 54
   ttttcctggg cttcatgggc catttaaatt gtgaaccccc agggacacag tgcttagccc      60
   aagccatcag tgcttgaaaa taggcatttc tgtcatcgag wttctgtatg ttgagatata     120
   ttttgagata ggcttaaggg tgaggacatt tggctggagt cagcaataag ttgcaa         176
<210> 55
   <211> 201
   <212> DNA
   <213> Homo sapiens
<400> 55
   cacaagacta gattgcagga agatgtgaaa gcatttggct aattctgaca tttggctaat      60
   aacatcctca cagtatgtcc caagtatctt ctgcccttcc yaccacggtg agcagtcatg     120
   acagtgaggg tggcataggt gggatgtgtg gctggcagaa gccagtgggc aagagttgtc     180
   cacagaatag tccatgagct t                                               201
<210> 56
   <211> 200
   <212> DNA
   <213> Homo sapiens
<400> 56
   tagagggggt gcgggacggg gactcacagg agatgcagga cggcccgaac atagtaattc       60
   ctggtaaagg gcccgaacag cttcaccacg gcggtcatgt rcttctgtcc cctgggggag     120
   ggaggaaggc gagacggcgc ggctgggcct ctcccactcg ggactccttt gctgccctgc     180
   tgaccacccc agggcaccca                                                 200
<210> 57
   <211> 201
   <212> DNA
   <213> Homo sapiens
<400> 57
   gttcacttgc tgctaccctc ttgtgcccac tttggcttaa taaatcccaa tccagcctag 60
   ctgatttact gaagaacaaa gggatgacta gtttttgcta ygccaaggtg agtctaggat 120
   cttaaaactt ttctaagggt gccaaggagt tagaatcagg ggacctaagt tttttctcct 180
   gaaggcattt ctgagtagta g 201
<210> 58
   <211> 201
   <212> DNA
   <213> Homo sapiens
<400> 58
   tgttgatgag aaaattactt tcccttctga cattgatcct caagttttct atgaactacc 60
   agaagcagta caaaaggaac tgctggcaga gtggaagaga rcaggatcag atttccacat 120
   tggacataaa taagcatatt cagcaaaaag gtctgaaaag caagggaata ccattatttt 180
   cggattagcg gtttattaag c 201
<210> 59
   <211> 201
   <212> DNA
   <213> Homo sapiens
<400> 59
   atgtgcatgt agccctttca gtaaggagtg tatggggagc ttgggccctc tgtggccttc 60
   catttatttg catgtagcct cctagtcaac tagggttgta mggggagcct atgtagtccc 120
   tctttggttc tctcatttcc aaatctcctg gtgacatatc tggctggtcc actgatctgt 180
   ccttcacccg aaccacgact g 201
<210> 60
   <211> 201
   <212> DNA
   <213> Homo sapiens
<400> 60
   gcacgtgggc ctccacgatt ccttggcatg cgtgtcactc tgggcaacag gctggtcggc 60
   cacgactgcg caggcgagag gcacagagcg actggagact ktagtccccc ggtttccctg 120
   gagaccaggc ggaagcggcc ggaagtagcg ctgcggttgg cagcggcggg atggagaagc 180
   tgggggtgga gccggaggag g 201
<210> 61
   <211> 30
   <212> DNA
   <213> Artificial Sequence
<220>
   <223> primer
<400> 61
   acgttggatg ccaagtaggg aatcaggatc 30
<210> 62
   <211> 30
   <212> DNA
   <213> Artificial Sequence
<220>
   <223> Homo sapiens
<400> 62
   acgttggatg ggtctccact gatatttggg 30
<210> 63
   <211> 24
   <212> DNA
   <213> Artificial Sequence
<220>
   <223> primer
<400> 63
   ggatctttca tctcattatt aggt 24
<210> 64
   <211> 30
   <212> DNA
   <213> Artificial Sequence
<220>
   <223> primer
<400> 64
   acgttggatg gttagaaagc agcactgtgg 30
<210> 65
   <211> 30
   <212> DNA
   <213> Artificial Sequence
<220>
   <223> primer
<400> 65
   acgttggatg tcttaggagg tcattggtgc 30
<210> 66
   <211> 17
   <212> DNA
   <213> Artificial Sequence
<220>
   <223> primer
<400> 66
   tggcagctct cagaagg 17
<210> 67
   <211> 31
   <212> DNA
   <213> Artificial Sequence
<220>
   <223> primer
<400> 67
   acgttggatg gtaggattct accttagaag c 31
<210> 68
   <211> 30
   <212> DNA
   <213> Artificial Sequence
<220>
   <223> primer
<400> 68
   acgttggatg taaggagttt cagtgcaccc 30
<210> 69
   <211> 17
   <212> DNA
   <213> Artificial Sequence
<220>
   <223> primer
<400> 69
   accttagaag cttgggg 17
<210> 70
   <211> 30
   <212> DNA
   <213> Artificial Sequence
<220>
   <223> primer
<400> 70
   acgttggatg ttccagtcct agtgaaacgg 30
<210> 71
   <211> 30
   <212> DNA
   <213> Artificial Sequence
<220>
   <223> primer
<400> 71
   acgttggatg accctggtgt ctgacttttg 30
<210> 72
   <211> 19
   <212> DNA
   <213> Artificial Sequence
<220>
   <223> primer
<400> 72
   aacgggagag aaagaaggt 19
<210> 73
   <211> 31
   <212> DNA
   <213> Artificial Sequence
<220>
   <223> primer
<400> 73
   acgttggatg cacagttgta aagactttgc c 31
<210> 74
   <211> 30
   <212> DNA
   <213> Artificial Sequence
<220>
   <223> primer
<400> 74
   acgttggatg gtggtttgat aatttccaag 30
<210> 75
   <211> 20
   <212> DNA
   <213> Artificial Sequence
<220>
   <223> primer
<400> 75
   tgcctaattt ctggcaacta 20
<210> 76
   <211> 31
   <212> DNA
   <213> Artificial Sequence
<220>
   <223> primer
<400> 76
   acgttggatg gatggactta agagatcatc c 31
<210> 77
   <211> 30
   <212> DNA
   <213> Artificial Sequence
<220>
   <223> primer
<400> 77
   acgttggatg tgaacatcag cagctttccg 30
<210> 78
   <211> 17
   <212> DNA
   <213> Artificial Sequence
<220>
   <223> primer
<400> 78
   agagatcatc ccaacct 17
<210> 79
   <211> 30
   <212> DNA
   <213> Artificial Sequence
<220>
   <223> primer
<400> 79
   acgttggatg acacttgttt gatgcctgcc 30
<210> 80
   <211> 30
   <212> DNA
   <213> Artificial Sequence
<220>
   <223> primer
<400> 80
   acgttggatg tttacagact gtcctctgcg 30
<210> 81
   <211> 18
   <212> DNA
   <213> Artificial Sequence
<220>
   <223> primer
<400> 81
   ctgcctctag ggcaatga 18
<210> 82
   <211> 30
   <212> DNA
   <213> Artificial Sequence
<220>
   <223> primer
<400> 82
   acgttggatg agactgcgag tcacgtgcga 30
<210> 83
   <211> 30
   <212> DNA
   <213> Artificial Sequence
<220>
   <223> primer
<400> 83
   acgttggatg gctttgtttt ctccacccac 30
<210> 84
   <211> 17
   <212> DNA
   <213> Artificial Sequence
<220>
   <223> primer
<400> 84
   gacagaggag gcggagc 17
<210> 85
   <211> 30
   <212> DNA
   <213> Artificial Sequence
<220>
   <223> primer
<400> 85
   acgttggatg aagcctttct taagctctgg 30

<210> 86
   <211> 30
   <212> DNA
   <213> Artificial Sequence
<220>
   <223> primer
<400> 86
   acgttggatg cttcctcatc tctctttccc 30
<210> 87
   <211> 21
   <212> DNA
   <213> Artificial Sequence
<220>
   <223> primer
<400> 87
   agctctggta acttgcatat a 21
<210> 88
   <211> 30
   <212> DNA
   <213> Artificial Sequence
<220>
   <223> primer
<400> 88
   acgttggatg tttcaagcca ccattgcagc 30
<210> 89
   <211> 30
   <212> DNA
   <213> Artificial Sequence
<220>
   <223> primer
<400> 89
   acgttggatg tcaaagtctg tgtgctcatg 30
<210> 90
   <211> 17
   <212> DNA
   <213> Artificial Sequence
<220>
   <223> primer
<400> 90
   ggtttgtctg aggcacc 17
<210> 91
   <211> 30
   <212> DNA
   <213> Artificial Sequence
<220>
   <223> primer
<400> 91
   acgttggatg ctcattagac tcagaaggcg 30
<210> 92
   <211> 30
   <212> DNA
   <213> Artificial Sequence
<220>
   <223> primer
<400> 92
   acgttggatg ccatctctcc ttcctcttac 30
<210> 93
   <211> 19
   <212> DNA
   <213> Artificial Sequence
<220>
   <223> primer
<400> 93
   aggtcacctg agtaagcag 19
<210> 94
   <211> 30
   <212> DNA
   <213> Artificial Sequence
<220>
   <223> primer
<400> 94
   acgttggatg aatggcctgt taaacctagc 30
<210> 95
   <211> 32
   <212> DNA
   <213> Artificial Sequence
<220>
   <223> primer
<400> 95
   acgttggatg gcatatcaag aaataaaaga cc 32
<210> 96
   <211> 23
   <212> DNA
   <213> Artificial Sequence
<220>
   <223> primer
<400> 96
   gctaaaacta agtgattttt ttg 23
<210> 97
   <211> 30
   <212> DNA
   <213> Artificial Sequence
<220>
   <223> primer
<400> 97
   acgttggatg tttctgaggg caagaacctg 30
<210> 98
   <211> 30
   <212> DNA
   <213> Artificial Sequence
<220>
   <223> primer
<400> 98
   acgttggatg tggagaagtt tcttaggtgg 30
<210> 99
   <211> 21
   <212> DNA
   <213> Artificial Sequence
<220>
   <223> primer
<400> 99
   ttacattctg aggaccatac a 21
<210> 100
   <211> 30
   <212> DNA
   <213> Artificial Sequence
<220>
   <223> primer
<400> 100
   acgttggatg tcaggctaaa ttgtcggctc 30
<210> 101
   <211> 31
   <212> DNA
   <213> Artificial Sequence
<220>
   <223> primer
<400> 101
   acgttggatg caaagacctg tgctaacatt c 31
<210> 102
   <211> 22
   <212> DNA
   <213> Artificial Sequence
<220>
   <223> primer
<400> 102
   gtcggctcta agaacatgaa gt 22
<210> 103
   <211> 30
   <212> DNA
   <213> Artificial Sequence
<220>
   <223> primer
<400> 103
   acgttggatg cagtgtacaa ctcttcacag 30
<210> 104
   <211> 30
   <212> DNA
   <213> Artificial Sequence
<220>
   <223> primer
<400> 104
   acgttggatg acagagacaa ggagagaagc 30
<210> 105
   <211> 20
   <212> DNA
   <213> Artificial Sequence
<220>
   <223> primer
<400> 105
   cttcacagag caatcgctca 20
<210> 106
   <211> 29
   <212> DNA
   <213> Artificial Sequence
<220>
   <223> primer
<400> 106
   acgttggatg ctccgcggtc atggagatc 29
<210> 107
   <211> 30
   <212> DNA
   <213> Artificial Sequence
<220>
   <223> primer
<400> 107
   acgttggatg ttacccagtt ctgcgcgtta 30
<210> 108
   <211> 17
   <212> DNA
   <213> Artificial Sequence
<220>
   <223> primer
<400> 108
   ctgtgcaaga aagtcaa 17
<210> 109
   <211> 30
   <212> DNA
   <213> Artificial Sequence
<220>
   <223> primer
<400> 109
   acgttggatg ggcaaggaac taagaggtag 30
<210> 110
   <211> 30
   <212> DNA
   <213> Artificial Sequence
<220>
   <223> primer
<400> 110
   acgttggatg acctgtaacc caggagaatg 30
<210> 111
   <211> 18
   <212> DNA
   <213> Artificial Sequence
<220>
   <223> primer
<400> 111
   tctagacgat ggaaactt 18
<210> 112
   <211> 30
   <212> DNA
   <213> Artificial Sequence
<220>
   <223> primer
<400> 112
   acgttggatg acagtagcca gtactcaatc 30
<210> 113
   <211> 30
   <212> DNA
   <213> Artificial Sequence
<220>
   <223> primer
<400> 113
   acgttggatg tggccttttg ccttatttcc 30
<210> 114
   <211> 17
   <212> DNA
   <213> Artificial Sequence
<220>
   <223> primer
<400> 114
   cagttcctaa ggcccaa 17
<210> 115
   <211> 30
   <212> DNA
   <213> Artificial Sequence
<220>
   <223> primer
<400> 115
   acgttggatg tcaagaaagt ctgggtcctc 30
<210> 116
   <211> 31
   <212> DNA
   <213> Artificial Sequence
<220>
   <223> primer
<400> 116
   acgttggatg ctcagattaa gatgtcttgg g 31
<210> 117
   <211> 17
   <212> DNA
   <213> Artificial Sequence
<220>
   <223> primer
<400> 117
   ctgggtcctc agtacta 17
<210> 118
   <211> 30
   <212> DNA
   <213> Artificial Sequence
<220>
   <223> primer
<400> 118
   acgttggatg ctagagaaac ccatagcaac 30
<210> 119
   <211> 30
   <212> DNA
   <213> Artificial Sequence
<220>
   <223> primer
<400> 119
   acgttggatg caagaggaag gacagatggc 30
<210> 120
   <211> 17
   <212> DNA
   <213> Artificial Sequence
<220>
   <223> primer
<400> 120
   ttgatggtca gtggtcc 17
<210> 121
   <211> 31
   <212> DNA
   <213> Artificial Sequence
<220>
   <223> primer
<400> 121
   acgttggatg gtgggaaatt gtactcattg g 31
<210> 122
   <211> 30
   <212> DNA
   <213> Artificial Sequence
<220>
   <223> primer
<400> 122
   acgttggatg tctggaaaat atatcgcctc 30
<210> 123
   <211> 20
   <212> DNA
   <213> Artificial Sequence
<220>
   <223> primer
<400> 123
   ttaaagcaaa ggttttgtct 20
<210> 124
   <211> 30
   <212> DNA
   <213> Artificial Sequence
<220>
   <223> primer
<400> 124
   acgttggatg acaggcacca acctctctag 30
<210> 125
   <211> 30
   <212> DNA
   <213> Artificial Sequence
<220>
   <223> primer
<400> 125
   acgttggatg ggagcctttc aaggaacctg 30
<210> 126
   <211> 20
   <212> DNA
   <213> Artificial Sequence
<220>
   <223> primer
<400> 126
   tagcacaggg gttcctccaa 20
<210> 127
   <211> 30
   <212> DNA
   <213> Artificial Sequence
<220>
   <223> primer
<400> 127
   acgttggatg gcttctagac gttgtttctg 30
<210> 128
   <211> 30
   <212> DNA
   <213> Artificial Sequence
<220>
   <223> primer
<400> 128
   acgttggatg tgctccattt tgccatcttc 30
<210> 129
   <211> 19
   <212> DNA
   <213> Artificial Sequence
<220>
   <223> primer
<400> 129
   tgtagatgta aaggctgtt 19
<210> 130
   <211> 30
   <212> DNA
   <213> Artificial Sequence
<220>
   <223> primer
<400> 130
   acgttggatg acagcaagtc cgcattcatc 30
<210> 131
   <211> 30
   <212> DNA
   <213> Artificial Sequence
<220>
   <223> primer
<400> 131
   acgttggatg caaactatgc tcttactagg 30
<210> 132
   <211> 23
   <212> DNA
   <213> Artificial Sequence
<220>
   <223> primer
<400> 132
   agcatcatct aagacataaa aag 23
<210> 133
   <211> 30
   <212> DNA
   <213> Artificial Sequence
<220>
   <223> primer
<400> 133
   acgttggatg gaatcagagg tcctagtcag 30
<210> 134
   <211> 30
   <212> DNA
   <213> Artificial Sequence
<220>
   <223> primer
<400> 134
   acgttggatg ttgactagat tcacggctcc 30
<210> 135
   <211> 17
   <212> DNA
   <213> Artificial Sequence
<220>
   <223> primer
<400> 135
   tcctagtcag ccttccc 17
<210> 136
   <211> 30
   <212> DNA
   <213> Artificial Sequence
<220>
   <223> primer
<400> 136
   acgttggatg gtgaggccgc tggttctaac 30
<210> 137
   <211> 30
   <212> DNA
   <213> Artificial Sequence
<220>
   <223> primer
<400> 137
   acgttggatg tgagcattaa accagggcag 30
<210> 138
   <211> 20
   <212> DNA
   <213> Artificial Sequence
<220>
   <223> primer
<400> 138
   ccgctggttc taaccaagag 20
<210> 139
   <211> 30
   <212> DNA
   <213> Artificial Sequence
<220>
   <223> primer
<400> 139
   acgttggatg tctatgtgcc cttctgcttc 30
<210> 140
   <211> 28
   <212> DNA
   <213> Artificial Sequence
<220>
   <223> primer
<400> 140
   acgttggatg gctgaaacag caagccca 28
<210> 141
   <211> 18
   <212> DNA
   <213> Artificial Sequence
<220>
   <223> primer
<400> 141
   ctctcacctg ggccaccc 18
<210> 142
   <211> 30
   <212> DNA
   <213> Artificial Sequence
<220>
   <223> primer
<400> 142
   acgttggatg atctccttct gtcccttctg 30
<210> 143
   <211> 30
   <212> DNA
   <213> Artificial Sequence
<220>
   <223> primer
<400> 143
   acgttggatg gaagccatgc tgttaacagt 30
<210> 144
   <211> 17
   <212> DNA
   <213> Artificial Sequence
<220>
   <223> primer
<400> 144
   tgtcccttct gatggca 17
<210> 145
   <211> 30
   <212> DNA
   <213> Artificial Sequence
<220>
   <223> primer
<400> 145
   acgttggatg acgtaaacca gaactgaggc 30
<210> 146
   <211> 30
   <212> DNA
   <213> Artificial Sequence
<220>
   <223> primer
<400> 146
   acgttggatg ttcttcgcct tgcaatccag 30
<210> 147
   <211> 20
   <212> DNA
   <213> Artificial Sequence
<220>
   <223> primer
<400> 147
   tcagaggctg aggcggggag 20
<210> 148
   <211> 30
   <212> DNA
   <213> Artificial Sequence
<220>
   <223> primer
<400> 148
   acgttggatg tcactttagg ccaactgctc 30
<210> 149
   <211> 30
   <212> DNA
   <213> Artificial Sequence
<220>
   <223> primer
<400> 149
   acgttggatg tttttaccca gccaggtctc 30
<210> 150
   <211> 17
   <212> DNA
   <213> Artificial Sequence
<220>
   <223> primer
<400> 150
   agaaactgga cccagtg 17
<210> 151
   <211> 30
   <212> DNA
   <213> Artificial Sequence
<220>
   <223> primer
<400> 151
   acgttggatg cctcatattt acgggagggc 30
<210> 152
   <211> 30
   <212> DNA
   <213> Artificial Sequence
<220>
   <223> primer
<400> 152
   acgttggatg aggcctgcca gaattgaaac 30
<210> 153
   <211> 17
   <212> DNA
   <213> Artificial Sequence
<220>
   <223> primer
<400> 153
   gagggcttca aaagtaa 17
<210> 154
   <211> 30
   <212> DNA
   <213> Artificial Sequence
<220>
   <223> primer
<400> 154
   acgttggatg aatgtaggat gccccagatg 30
<210> 155
   <211> 30
   <212> DNA
   <213> Artificial Sequence
<220>
   <223> primer
<400> 155
   acgttggatg cagctgctgt gagttagttc 30
<210> 156
   <211> 23
   <212> DNA
   <213> Artificial Sequence
<220>
   <223> primer
<400> 156
   ttgtcagaaa tgaaaaatac atc 23
<210> 157
   <211> 30
   <212> DNA
   <213> Artificial Sequence
<220>
   <223> primer
<400> 157
   acgttggatg tgtttacttt gccaccaacc 30
<210> 158
   <211> 30
   <212> DNA
   <213> Artificial Sequence
<220>
   <223> primer
<400> 158
   acgttggatg agtgccaaac ctacagaagc 30
<210> 159
   <211> 17
   <212> DNA
   <213> Artificial Sequence
<220>
   <223> primer
<400> 159
   tgccaccaac ctgccct 17
<210> 160
   <211> 30
   <212> DNA
   <213> Artificial Sequence
<220>
   <223> primer
<400> 160
   acgttggatg cattggcaaa gtccagtcac 30
<210> 161
   <211> 30
   <212> DNA
   <213> Artificial Sequence
<220>
   <223> primer
<400> 161
   acgttggatg agtctaagta cccactcagc 30
<210> 162
   <211> 17
   <212> DNA
   <213> Artificial Sequence
<220>
   <223> primer
<400> 162
   agtcaccaaa gcagttt 17
<210> 163
   <211> 30
   <212> DNA
   <213> Artificial Sequence
<220>
   <223> primer
<400> 163
   acgttggatg atgagtgccg ggctccagaa 30
<210> 164
   <211> 30
   <212> DNA
   <213> Artificial Sequence
<220>
   <223> primer
<400> 164
   acgttggatg agaaggagga acagaggcac 30
<210> 165
   <211> 17
   <212> DNA
   <213> Artificial Sequence
<220>
   <223> primer
<400> 165
   cttcccttct tgggatt 17
<210> 166
   <211> 30
   <212> DNA
   <213> Artificial Sequence
<220>
   <223> primer
<400> 166
   acgttggatg tctttgctct tccactccag 30
<210> 167
   <211> 30
   <212> DNA
   <213> Artificial Sequence
<220>
   <223> primer
<400> 167
   acgttggatg gaaaagggac aagaggaacg 30
<210> 168
   <211> 20
   <212> DNA
   <213> Artificial Sequence
<220>
   <223> primer
<400> 168
   ccactccagt ccttaagcga 20
<210> 169
   <211> 30
   <212> DNA
   <213> Artificial Sequence
<220>
   <223> primer
<400> 169
   acgttggatg tgagatctcg ggaaagagtc 30
<210> 170
   <211> 30
   <212> DNA
   <213> Artificial Sequence
<220>
   <223> primer
<400> 170
   acgttggatg aaaccagagg tgacttttcc 30
<210> 171
   <211> 24
   <212> DNA
   <213> Artificial Sequence
<220>
   <223> primer
<400> 171
   aaagagtctc tttataaata tggg 24
<210> 172
   <211> 30
   <212> DNA
   <213> Artificial Sequence
<220>
   <223> primer
<400> 172
   acgttggatg tggataaggt cacctgatgc 30
<210> 173
   <211> 30
   <212> DNA
   <213> Artificial Sequence
<220>
   <223> primer
<400> 173
   acgttggatg agtgtgtgca ggttgtagtc 30
<210> 174
   <211> 17
   <212> DNA
   <213> Artificial Sequence
<220>
   <223> primer
<400> 174
   tacacacagc tccgaat 17
<210> 175
   <211> 30
   <212> DNA
   <213> Artificial Sequence
<220>
   <223> primer
<400> 175
   acgttggatg ggtttagatg gaaggaacgg 30
<210> 176
   <211> 30
   <212> DNA
   <213> Artificial Sequence
<220>
   <223> primer
<400> 176
   acgttggatg cagctctgtc tgataaaggg 30
<210> 177
   <211> 20
   <212> DNA
   <213> Artificial Sequence
<220>
   <223> primer
<400> 177
   aagtggcgtg tatgtgtaat 20
<210> 178
   <211> 30
   <212> DNA
   <213> Artificial Sequence
<220>
   <223> primer
<400> 178
   acgttggatg ccctcatgtt actaacaggc 30
<210> 179
   <211> 30
   <212> DNA
   <213> Artificial Sequence
<220>
   <223> primer

<400> 179
   acgttggatg gcagcagcct ttaatgcatc 30
<210> 180
   <211> 20
   <212> DNA
   <213> Artificial Sequence
<220>
   <223> primer
<400> 180
   gttactaaca ggctgtaact 20
<210> 181
   <211> 30
   <212> DNA
   <213> Artificial Sequence
<220>
   <223> primer
<400> 181
   acgttggatg gtctataaag cagacttccc 30
<210> 182
   <211> 30
   <212> DNA
   <213> Artificial Sequence
<220>
   <223> primer
<400> 182
   acgttggatg gcacaatgtc tgacatctgg 30
<210> 183
   <211> 19
   <212> DNA
   <213> Artificial Sequence
<220>
   <223> primer
<400> 183
   agacttccca tggatgttt 19
<210> 184
   <211> 30
   <212> DNA
   <213> Artificial Sequence
<220>
   <223> primer
<400> 184
   acgttggatg aatgtcatca gtcaaggccc 30
<210> 185
   <211> 30
   <212> DNA
   <213> Artificial Sequence
<220>
   <223> primer
<400> 185
   acgttggatg tgatctcagt gacgatgcag 30
<210> 186
   <211> 17
   <212> DNA
   <213> Artificial Sequence
<220>
   <223> primer
<400> 186
   tgaggcctgg ggagacc 17
<210> 187
   <211> 30
   <212> DNA
   <213> Artificial Sequence
<220>
   <223> primer
<400> 187
   acgttggatg ggaaagatat agccaccagg 30
<210> 188
   <211> 30
   <212> DNA
   <213> Artificial Sequence
<220>
   <223> primer
<400> 188
   acgttggatg aatggaagtc ctccatcagc 30
<210> 189
   <211> 18
   <212> DNA
   <213> Artificial Sequence
<220>
   <223> primer
<400> 189
   caggagtagt tatgagga 18
<210> 190
   <211> 30
   <212> DNA
   <213> Artificial Sequence
<220>
   <223> primer
<400> 190
   acgttggatg cacagtgaga aaaggaagag 30
<210> 191
   <211> 30
   <212> DNA
   <213> Artificial Sequence
<220>
   <223> primer
<400> 191
   acgttggatg tgcctgttgc caaatgcttg 30
<210> 192
   <211> 22
   <212> DNA
   <213> Artificial Sequence
<220>
   <223> primer
<400> 192
   aaggaagaga aagataaaat ca 22
<210> 193
   <211> 30
   <212> DNA
   <213> Artificial Sequence
<220>
   <223> primer
<400> 193
   acgttggatg gtaagcttgg ctctgtttcc 30
<210> 194
   <211> 30
   <212> DNA
   <213> Artificial Sequence
<220>
   <223> primer
<400> 194
   acgttggatg tgaaatccca aaagcccagg 30
<210> 195
   <211> 23
   <212> DNA
   <213> Artificial Sequence
<220>
   <223> primer
<400> 195
   gctctgtttc caaactcata aaa 23
<210> 196
   <211> 31
   <212> DNA
   <213> Artificial Sequence
<220>
   <223> primer
<400> 196
   acgttggatg gtcaattggc ctaattttcc c 31
<210> 197
   <211> 30
   <212> DNA
   <213> Artificial Sequence
<220>
   <223> primer
<400> 197
   acgttggatg gagtggaatt gttccaggtc 30
<210> 198
   <211> 22
   <212> DNA
   <213> Artificial Sequence
<220>
   <223> primer
<400> 198
   cattttaatt tctccagagc tt 22
<210> 199
   <211> 30
   <212> DNA
   <213> Artificial Sequence
<220>
   <223> primer
<400> 199
   acgttggatg caaacaggaa ccagaccatc 30
<210> 200
   <211> 30
   <212> DNA
   <213> Artificial Sequence
<220>
   <223> primer
<400> 200
   acgttggatg agcaacctca cattgacacg 30
<210> 201
   <211> 17
   <212> DNA
   <213> Artificial Sequence
<220>
   <223> primer
<400> 201
   accagaccat cagatcc 17
<210> 202
   <211> 30
   <212> DNA
   <213> Artificial Sequence
<220>
   <223> primer
<400> 202
   acgttggatg aggtttggga acactggttg 30
<210> 203
   <211> 30
   <212> DNA
   <213> Artificial Sequence
<220>
   <223> primer
<400> 203
   acgttggatg ggaacgtttg tcctaatggg 30
<210> 204
   <211> 20
   <212> DNA
   <213> Artificial Sequence
<220>
   <223> primer
<400> 204
   acaagccact gatgagggag 20
<210> 205
   <211> 30
   <212> DNA
   <213> Artificial Sequence
<220>
   <223> primer
<400> 205
   acgttggatg ttttctggtt acccaataag 30
<210> 206
   <211> 31
   <212> DNA
   <213> Artificial Sequence
<220>
   <223> primer
<400> 206
   acgttggatg caatgaccca agtatctaga g 31
<210> 207
   <211> 18
   <212> DNA
   <213> Artificial Sequence
<220>
   <223> primer
<400> 207
   atcatgtgac tttccaca 18
<210> 208
   <211> 30
   <212> DNA
   <213> Artificial Sequence
<220>
   <223> primer
<400> 208
   acgttggatg agtgcagaca ctatctgctc 30
<210> 209
   <211> 30
   <212> DNA
   <213> Artificial Sequence
<220>
   <223> primer
<400> 209
   acgttggatg aagtgatgca gcaatgctgg 30
<210> 210
   <211> 20
   <212> DNA
   <213> Artificial Sequence
<220>
   <223> primer
<400> 210
   cacctccccc actagtactc 20
<210> 211
   <211> 30
   <212> DNA
   <213> Artificial Sequence
<220>
   <223> primer
<400> 211
   acgttggatg agagtgcctg ctcagataac 30
<210> 212
   <211> 30
   <212> DNA
   <213> Artificial Sequence
<220>
   <223> primer
<400> 212
   acgttggatg tcaagaggta aaaggagctg 30
<210> 213
   <211> 17
   <212> DNA
   <213> Artificial Sequence
<220>
   <223> primer
<400> 213
   tgctcatccc tgggagt 17
<210> 214
   <211> 30
   <212> DNA
   <213> Artificial Sequence
<220>
   <223> primer
<400> 214
   acgttggatg gtagggactt tctttgctgg 30
<210> 215
   <211> 30
   <212> DNA
   <213> Artificial Sequence
<220>
   <223> primer
<400> 215
   acgttggatg tgtaaaaact ctgggaaggg 30
<210> 216
   <211> 20
   <212> DNA
   <213> Artificial Sequence
<220>
   <223> primer
<400> 216
   ctcatcccca agaattattt 20
<210> 217
   <211> 30
   <212> DNA
   <213> Artificial Sequence
<220>
   <223> primer
<400> 217
   acgttggatg ctgcggtatg agtctgtatc 30
<210> 218
   <211> 30
   <212> DNA
   <213> Artificial Sequence
<220>
   <223> primer
<400> 218
   acgttggatg ctctgtgctc caagtacaac 30
<210> 219
   <211> 21
   <212> DNA
   <213> Artificial Sequence
<220>
   <223> primer
<400> 219
   aacctttcac ttcccttcaa a 21
<210> 220
   <211> 30
   <212> DNA
   <213> Artificial Sequence
<220>
   <223> primer
<400> 220
   acgttggatg ttagcccaag ccatcagtgc 30
<210> 221
   <211> 30
   <212> DNA
   <213> Artificial Sequence
<220>
   <223> primer
<400> 221
   acgttggatg ctcaccctta agcctatctc 30
<210> 222
   <211> 17
   <212> DNA
   <213> Artificial Sequence
<220>
   <223> primer
<400> 222
   gcatttctgt catcgag 17
<210> 223
   <211> 30
   <212> DNA
   <213> Artificial Sequence
<220>
   <223> primer
<400> 223
   acgttggatg acatcctcac agtatgtccc 30
<210> 224
   <211> 30
   <212> DNA
   <213> Artificial Sequence
<220>
   <223> primer
<400> 224
   acgttggatg tcactgtcat gactgctcac 30
<210> 225
   <211> 20
   <212> DNA
   <213> Artificial Sequence
<220>
   <223> primer
<400> 225
   caagtatctt ctgcccttcc 20
<210> 226
   <211> 29
   <212> DNA
   <213> Artificial Sequence
<220>
   <223> primer
<400> 226
   acgttggatg aacagcttca ccacggcgg 29
<210> 227
   <211> 30
   <212> DNA
   <213> Artificial Sequence
<220>
   <223> primer
<400> 227
   acgttggatg aaaggagtcc cgagtgggag 30
<210> 228
   <211> 20
   <212> DNA
   <213> Artificial Sequence
<220>
   <223> primer
<400> 228
   cttcaccacg gcggtcatgt 20
<210> 229
   <211> 30
   <212> DNA
   <213> Artificial Sequence
<220>
   <223> primer
<400> 229
   acgttggatg taactccttg gcacccttag 30
<210> 230
   <211> 30
   <212> DNA
   <213> Artificial Sequence
<220>
   <223> primer
<400> 230
   acgttggatg ctgaagaaca aagggatgac 30
<210> 231
   <211> 17
   <212> DNA
   <213> Artificial Sequence
<220>
   <223> primer
<400> 231
   cctagactca ccttggc 17
<210> 232
   <211> 31
   <212> DNA
   <213> Artificial Sequence
<220>
   <223> primer
<400> 232
   acgttggatg gcttttcaga cctttttgct g 31
<210> 233
   <211> 30
   <212> DNA
   <213> Artificial Sequence
<220>
   <223> primer
<400> 233
   acgttggatg aaaaggaact gctggcagag 30
<210> 234
   <211> 18
   <212> DNA
   <213> Artificial Sequence
<220>
   <223> primer
<400> 234
   tgtggaaatc tgatcctg 18
<210> 235
   <211> 30
   <212> DNA
   <213> Artificial Sequence
<220>
   <223> primer
<400> 235
   acgttggatg tgagagaacc aaagagggac 30
<210> 236
   <211> 30
   <212> DNA
   <213> Artificial Sequence
<220>
   <223> primer
<400> 236
   acgttggatg ttgcatgtag cctcctagtc 30
<210> 237
   <211> 17
   <212> DNA
   <213> Artificial Sequence
<220>
   <223> primer
<400> 237
   gactacatag gctcccc 17
<210> 238
   <211> 30
   <212> DNA
   <213> Artificial Sequence
<220>
   <223> primer
<400> 238
   acgttggatg atgcgtgtca ctctgggcaa 30
<210> 239
   <211> 29
   <212> DNA
   <213> Artificial Sequence
<220>
   <223> primer
<400> 239
   acgttggatg tggtctccag ggaaaccgg 29
<210> 240
   <211> 17
   <212> DNA
   <213> Artificial Sequence
<220>
   <223> primer
<400> 240
   cagagcgact ggagact 17
<210> 241
   <211> 200
   <212> DNA
   <213> Homo sapiens
<400> 241
   ctataaatag ggcatcgtga cccggccagg ggaagaagct gccgttgttc tgggtactac 60
   agcagaaggt aagccggggg ccccctcagc tccttctcgg ycttgtctct ctcagatgta 120
   actgagctgt gggctaggag gaaaaggccg ggaggaggca cggtgatgac tgaaaaacct 180
   ctcccctctc ataagaccag 200
<210> 242
   <211> 200
   <212> DNA
   <213> Homo sapiens
<400> 242
   tgagagagac aaggccgaga aggagctgag ggggcccccg gcttaccttc tgctgtagta 60
   cccagaacaa cggcagcttc ttcccccggc cgggtcacga kgccctattt atagctgagg 120
   ggtggggatg gagctgttcc caggctgcct gtgcacaggc tggagaggag ggttacatca 180
   cttggccaga ccacaggctg 200
<210> 243
   <211> 200
   <212> DNA
   <213> Homo sapiens
<400> 243
   tgtagtgtct gtaatacctt agttttttcc acagatgctt gtgatttttg aacaatacgt 60
   gaaagatgca agcacctgaa tttctgtttg aatgcggaac matagctggt tatttctccc 120
   ttgtgttagt aataaacgtc ttgccacaat aagcctccaa aaattttatc tttcatttag 180
   cagccaaaca gatgtataca 200
<210> 244
   <211> 200
   <212> DNA
   <213> Homo sapiens
<400> 244
   ctcccacgct ctctggactt cacagaactg gatgttgctg ctgagaagat tgacaggttc 60
   atgcaggctg tgacaggatg gaagactggc tgctccctga ygggagccag tgtggacagc 120
   accctggctt tcaacaccta cgtccacttc caaggtaagg caaacctctc tgctggctct 180
   ggccctagga cttagtatcc 200
<210> 245
   <211> 30
   <212> DNA
   <213> Artificial Sequence
<220>
   <223> primer
<400> 245
   acgttggatg gcccacagct cagttacatc 30
<210> 246
   <211> 30
   <212> DNA
   <213> Artificial Sequence
<220>
   <223> primer
<400> 246
   acgttggatg actacagcag aaggtaagcc 30
<210> 247
   <211> 21
   <212> DNA
   <213> Artificial Sequence
<220>
   <223> primer
<400> 247
   gttacatctg agagagacaa g 21
<210> 248
   <211> 30
   <212> DNA
   <213> Artificial Sequence
<220>
   <223> primer
<400> 248
   acgttggatg tgtagtaccc agaacaacgg 30
<210> 249
   <211> 30
   <212> DNA
   <213> Artificial Sequence
<220>
   <223> primer
<400> 249
   acgttggatg agcctgggaa cagctccatc 30
<210> 250
   <211> 20
   <212> DNA
   <213> Artificial Sequence
<220>
   <223> primer
<400> 250
   ttcccccggc cgggtcacga 20
<210> 251
   <211> 30
   <212> DNA
   <213> Artificial Sequence
<220>
   <223> primer
<400> 251
   acgttggatg aggcttattg tggcaagacg 30
<210> 252
   <211> 30
   <212> DNA
   <213> Artificial Sequence
<220>
   <223> primer
<400> 252
   acgttggatg gtgaaagatg caagcacctg 30
<210> 253
   <211> 20
   <212> DNA
   <213> Artificial Sequence
<220>
   <223> Homo sapiens
<400> 253
   agggagaaat aaccagctat 20
<210> 254
   <211> 30
   <212> DNA
   <213> Artificial Sequence
<220>
   <223> primer
<400> 254
   acgttggatg gattgacagg ttcatgcagg 30
<210> 255
   <211> 30
   <212> DNA
   <213> Artificial Sequence
<220>
   <223> primer
<400> 255
   acgttggatg acgtaggtgt tgaaagccag 30
<210> 256
   <211> 21
   <212> DNA
   <213> Artificial Sequence
<220>
   <223> primer
<400> 256
   ggaagactgg ctgctccctg a 21